# EUROPEAN PATENT APPLICATION

(11) **EP 3 888 680 A2**
(43) Date of publication of application: **06.10.2021**
(21) Application number: 19889322.4
(22) Date of filing: 25.11.2019
(51) Int. Cl.: A61K 45/00, A61K 33/00, A61K 31/00, A23L 33/16, A61P 37/02, A23L 33/10, A61P 37/06

(54) **SUBSTANCE FOR TREATING AND/OR PREVENTING ALLERGIC DISEASES, AND DESIGN METHOD AND PREPARATION METHOD THEREFOR**

(30) Priority: 28.11.2018 CN 201811437559; 25.08.2019 CN 201910787182
(71) Applicant: Suzhou Zhihe Structural Medicine Technology Co., Ltd., Suzhou, Jiangsu 215123 (CN)
(72) Inventor: DONG, Futian, Suzhou, Jiangsu 215123 (CN)
(74) Representative: Bianchetti & Minoja SRL
(86) International application number: PCT/CN2019/120727
(87) International publication number: WO 2020/108448

(57) **Abstract**

The present invention discloses a substance for treating and/or preventing allergic diseases and a design method and preparation method thereof. The stable structure corresponding to the substance related to the allergic disease is used as the substance for treating and/or preventing the allergic disease. The essence of disease is the imbalance of biological structure system. The imbalance of the structure of the substance in the biological structure system will cause diseases related to said substance. The present disclosure relates to the treatment and/or prevention of the allergic disease by recognizing the stable structure corresponding to the substance by the body, adjusting the expression of its gene expression or genes by the self-adaptation, self-organization and other functions of body's structural system, and restoring the balance of the structure of the substance in the biological structure system.

## Description

### Technical Field

The invention belongs to the fields of medicines, health-care foods, food additives and the like, and particularly relates to substance for treating and/or preventing allergic diseases and preparation method thereof.

### Background

Allergic disease is a pathological immune disease that causes tissue damage or dysfunction due to antigenic stimulation of the body, and mainly comprises food allergy, allergic rhinitis, allergic dermatitis, asthma, anaphylactic shock and the like. With the development of society and economy, changes in dietary structure and living environment, the incidence of allergic diseases in the world is increasing year by year, and the allergic disease has been listed by the World Health Organization as one of the three major diseases that need to be studied and prevented in the 21st century. Epidemiological survey report of the World Allergy Organization shows that the proportion of allergic patients worldwide has reached 22%.

In the face of such raging allergic diseases, the existing treatment methods are extremely deficient. At present, medical treatment of allergies mainly includes drug treatment represented by antihistamines, and desensitization therapy. These drugs are aimed at inhibiting allergic reactions in different stages of allergies, and are symptomatic treatments with definite curative effect. However, the recurrence rate after drug withdrawal is high, and there are many adverse reactions. Desensitization therapy, also known as specific immunotherapy, is the only cause-specific treatment that can change the natural course of allergic diseases. Desensitization therapy is to gradually increase the intensity of exposure of the patient to the allergen after the allergen is clinically determined, including increasing the dose, concentration and contact time of the allergen, so that the body can develop tolerance, and when it comes into contact with the allergen again after desensitization therapy, allergies will no longer occur, or allergic symptoms will be alleviated. The duration of desensitization therapy is proportional to the effective rate, and the desensitization therapy has the characteristics of slow onset and longer course of treatment. It usually takes 3-5 years and will produce allergic side effects during the treatment, which will decrease the patient's compliance and make it difficult to persist in completing the entire course of treatment.

According to the new medical model, namely structural information medicine, the present invention recognizes life from the perspective of structural system, and restores the homeostasis of structural system by allowing the body to recognize the information of specific structure, using the self-adaptive and self-organizing functions of the structural system of the body, to achieve the purpose of preventing and curing diseases.

### 1. The Medical Model of Traditional Chinese Medicine (TCM) and Western Medicine

The existing medical system mainly comprises Western medicine and traditional Chinese medicine, wherein the Western medicine is based on microscopic local structure, which is good at studying things, but weak in making a thorough inquiry into truth; on the contrary, the traditional Chinese medicine is mainly based on the macroscopic overall function system, which is good at the theory of the things but weak in the aspect of studying things. The advantages of the two are complementary, but the theoretical system and the research method are opposite to each other. Western medicine mainly relies on scientific experiments, using basic research methods such as anatomical physiology, biochemistry and molecular biology, and clinical trials to analyze the essence of disease through the theory of segmentation and reduction. Therefore, Western medicine focuses more on the structural materials of pathological changes, analyzes the change of specific structures, and then directly eliminate the surplus or supplement the deficiency from the level of local material structures by means of chemical drugs, operations, rays and the like, such as H2 receptor antagonists for treating hyperchlorhydria, hormone replacement therapy and the like. Such therapy has a fast onset and is effective in some acute diseases, surgical diseases and infectious diseases. However, Western medicine lacks a comprehensive understanding of the body. This type of treatment can only temporarily solve the problem of local structure. Long-term use will have adverse effects on other structures of the body and disrupt the homeostasis of the body. In fact, from 2010, one third of the diseases in the world are drug-induced diseases, which are all caused by inevitable side effects of drugs. Moreover, Western medicine has little effect on chronic degenerative diseases that have complex pathogenic factors and are affected by multiple factors, such as cardiovascular and cerebrovascular diseases, multiple sclerosis, senile dementia and the like, and is difficult to suppress the increasing morbidity and mortality of these diseases.

TCM treats life as a whole, unifying the human body with society, psychology, and environment. The core of TCM is syndrome differentiation and treatment, which reflects the objective laws of nature with humans, health and disease. Health in the meaning of TCM emphasizes the relative balance and coordination of Yin and Yang in the body in subjective and objective environments, i.e., the balance of functional systems. When this balance is broken and the order and homeostasis of the body is disturbed or destroyed, i.e., the functional system of the body is unbalanced, diseases occur. TCM diagnoses the nature of diseases by inquiring a large amount of functional system information, and then mobilizes the functional systems of the human body by methods of medicines, acupuncture, massage and the like, so as to eliminate the surplus, supplement the deficiency, strengthen the body resistance to eliminate pathogenic factors, and restore the body's homeostasis. TCM therapy has a slow onset of action, but it reduces the side effects of replacement therapy or antagonistic drugs, and is quite effective in disease prevention and treatment, health preservation, rehabilitation and the like.

In summary, Western medicine focuses more on micro structural balance, and TCM focuses more on macro functional system balance, both of which aim to correct the imbalance of the body at different levels. However, the two are completely different in the epistemology and methodology of disease and health. In order to allow these two medical models to complement each other, we need to combine the two with a more inclusive perspective and theory, and develop more effective substances for treating diseases, so as to make more outstanding contributions to human health.

### 2. Structural Information Medicine

### (1) Basic Theory of Combining Western Medicine and TCM by Structural System

After the Big Bang, positive and negative charges appeared, and the two interacted to form a simple structure of hydrogen, and then the interaction between the structures formed a more complex structure, and finally formed a macroscopic matter, including the entire biological world. Therefore, everything in the universe is unified in structure. The substance, energy and information constituting all things in the universe are also unified in the structure. Firstly, different structures form different substances, the substances are specific structures and are the basis for realizing functions. The structure and function of the living system are matched, and the specific structure has the specific function. The change of the specific structure will naturally affect the specific function of the corresponding system. Secondly, the bond energy of the interaction between structures is a relatively abstract structure, indicating the stability of the structure, and the energy is the internal driving force for the interconnection of the structure. Finally, the relationship and the order among the structures are information, the information is the most abstract structure among the three (i.e., substance, energy and information), plays a role in combining and controlling substances and energy, reflects the intrinsic rules and essence of the structures, just as the essence of organisms is determined by its abstract structure, gene. Therefore, the structure is a unity of substance, energy, and information, that is, a unity of a concrete structure and an abstract structure.

A living being is an organic system, essentially an organic structural system dominated by abstract structures. The structural system is an organic structure whole with a certain function formed by relatively stable connection ways, organizational order and temporal and spatial relations among several elements. Any element itself is also a structural system, a subsystem that constitutes the original system, and the subsystem *per se* must be composed of sub-level subsystems. The structural system of a living being is composed of structural subsystems at different levels, such as immune system, gastrointestinal system and the like, and the living being itself is a subsystem of a larger structural system, e.g., nature, and maintains its own existence and reproduction in the process of continuous exchange of substance, energy and information with nature.

DNA, as the genetic material of an organism, stores all the information during life activities, including growth, development, aging and death. In the theory of biochemical individualization, Dr. Roger Williams showed that the difference in the biochemical composition of the human body comes from the difference in genes and gene expression, that is, the difference in the spatial structure of molecules resulting from gene expression. Dr. Susumu Tonegawa, Nobel Prize winner in Physiology, showed that all diseases except trauma are related to genes. The causes of the disease mainly include two aspects: one is that the structures that constitute the body are damaged or changed by the structural environment; the other is improper gene expression. The fundamental cause of structural imbalance is the change of abstract structure, that is, the change of gene expression, such as abnormal histone acetylation, methylation, and abnormal gene methylation. If the specific structure has changed, as long as the genes related to the structure can be expressed normally, the body's metabolism will gradually replace the diseased structure with the normal structure, and the body can return to a healthy state. Therefore, the core of biological health lies in the genes and their expression.

Western medicine studies the specific structural changes, takes the disease as a model, and treats the disease based on the model. Western medicine focuses on the study of local tissues and functions in structural systems, can detect with the aid of instruments and go deep into specific and microscopic material structures. Western medicine focuses on and emphasizes the existence forms of specific human material structures and morphologies, such as anatomy, molecular biology, cytology, histology, etc., all of which are experimental ways to study and observe specific structures in structural systems from different angles, and are deficient in abstract thinking, ignoring the integrity of the body as a structural system. Therefore, it is easy to have limitations in understanding and practice. Western medicine mainly directly intervenes in specific structures, uses drugs to change and influence local structures of the body to achieve a new balance, including the study of genes, and tends to directly change genes or gene expression. TCM takes humans as a model, studies the functions of various organs of the body and the interrelationships and effects between functions under the guidance of holism. TCM does not detect specific material structures, but diagnoses diseases through functional manifestations and external information. However, the function is determined by the structure, the essence of the functional system is the structural system, and the mutual generation and restriction between the functional subsystems are essentially the mutual promotion and mutual restriction between the structural subsystems. TCM achieves overall balance by conditioning the functional order and strength of the body, and the essence of the TCM is to restore the dynamic balance of the structural system. However, TCM has great limitation due to the lack of in-depth understanding of the specific body structure. Western medicine mainly builds disease models from the perspective of microstructure, while TCM builds life models from the perspective of macroscopic functions. The microstructure of Western medicine has found the material basis for the TCM, that is, TCM syndromes have material support, namely the structural support of gene expression, and the essence is the microcosmic structural imbalance. In the structural system, based on the unification of the micro and macro, the part and the whole as well as the concrete and the abstract, the local and microcosmic concrete structure can be utilized to convert the concrete structure into abstract structural information through the recognition of the concrete structure by the body, stimulate the body's self-adaptive and self-organizing functions to adjust the genes or the expression of the genes, and regain the balance of the structural system.

As a system, the structural system has the basic characteristics of the system, such as integrity, openness, stability, and self-organization. Relative to the environment, the system is the unity of closedness and openness. The interaction between the structures in the structural system is on the premise of the interaction between the structures and the environment and on the condition of the external environment of the structures. The evolution of the same structural system depends on the external structure. The proper change and adjustment of gene expression along with changes in external structure is the basic function of organisms to adapt to the environment, maintain survival, and maintain shelf health. If this function is lacking, the organisms will generally be eliminated. The structural system of the human body has this powerful self-health function. Structural information medicine is a relatively complex, highly ordered, and autonomously regulated mechanism of action that mobilizes and regulates different levels of subsystems in the structural system, including genes, molecules, cells, tissues and organs, through the structural information of specific structures, so as to restore the structural balance of the body and achieve the purpose of preventing and treating diseases. Structural information medicine combines the theory of Western medicine that takes specific structure and function as the research object, and the theory of TCM that restores balance by adjusting the body itself.

### (2) Problems Solved by Structural Information Medicine

Living organisms cannot exist independently from the environment. The external structural environment interacts with the body's structural system through structure, namely matter, energy and information. Each individual is a unique structural system resulting from the interaction of genes and environment. Structural information medicine emphasizes the biological internal structural system and its dynamic balance with the external structural environment. It is this dynamic balance that interprets the process of life. In addition to restoring the dynamic balance of the body's own structural system and increasing the body's resistance, structural information medicine also focuses on symbiosis and co-progress. Symbiosis means that the body no longer resists and defends the external structure, but continuously internalizes the external structure information, and realizes the balance between the biological body's structural system and the external structure through the self-adaptation and self-organization functions of the organism, and realizes the transition from confrontation to peaceful coexistence and even benefit from it. Co-progress means that the structural system of a biological organism obtains the structural characteristics of other biological organisms that are beneficial to the health, survival and reproduction through structural information. The present invention is based on the established theoretical system of structural information medicine, uses the stable structure corresponding to the substance to regulate the gene or the expression of the gene through the self-adapting, self-organizing and other abilities of the organism's own structural system, so as to treat the diseases related to the substance.

### Disclosure of Invention

The object of the present invention is to address the above-mentioned drawbacks of the prior art by providing a method for designing a substance for the treatment and/or prevention of allergic diseases.

Another object of the invention is to provide a substance designed according to this method.

The invention also aims to provide a preparation method and application of the substance.

The structural system of a biological organism is an organic structural system that combines stability and adaptability. It can adapt to the environment, maintain a dynamic balance between itself and the external environment, and then achieve better survival and reproduction. The adaptability of the biological structure system is based on the stability of the biological structure, and the stable structure is the dominant structure that plays a key role.

Biological structure system is an organic giant system formed by spiral development of core structure composed of concrete structure and abstract structure. The core structure of the biological structural system, that is, a complex in which a concrete structure and an abstract structure are unified in a nucleic acid-protein structural system, serves as a genetic information carrier and expression system, and plays a dominant role in the function of the biological structural system. What plays a dominant role in the core structure is the stable structure. At the atomic level, the abstract structure DNA and the concrete structure protein in the core structure are both biological macromolecules based on carbon elements. There are 4 electrons in the outermost layer of a carbon atom, and it is neither easy to lose electrons nor get electrons easily. Such structure is favorable for the carbon atom to form 4 covalent bonds. The covalent bond has high stability and is the strongest acting force among organic molecules. The bond energy of the covalent single bond mainly formed by carbon atoms is about 300-500 KJ/moL, and the thermal cracking temperature is 500-800°C.

The primary structure of DNA determines the double-helix secondary structure of DNA through the actions of base hydrogen bond, base stacking force and the like, namely, the stable primary structure determines the adaptive secondary structure of the DNA, and the core of genetic information is in the stable structure. DNA transfers genetic information to RNA by transcription and finally RNA expresses genetic information into protein by translation. This is the law followed by all cellular organisms. The establishment of the central law clarifies the information transmission path of genetic information from DNA to RNA to protein. From the base sequence in the primary structure of DNA to the base sequence in the primary structure of RNA and then to the amino acid sequence in the primary structure of protein, the genetic information carrier maps the information one-to-one to the functional information carrier amino acids through base complementary pairing and triplet codons. Therefore, this information transmission path is based on the stable structure of these biological macromolecules, and is transferred from the stable structure of the abstract structure to the stable structure of the concrete structure.

The covalent backbone in the primary structure of a protein contains hundreds of single bonds which can rotate freely, but the rotational freedom of each single bond is limited by factors such as the rigid planar nature of the peptide bonds in the primary structure, the number and position of hydrophilic/hydrophobic amino acids and acidic/basic amino acids, and the like, and finally, a three-dimensional structure which is most thermodynamically stable is formed. Therefore, in fact, the control component of the final structure of the protein, that is, the amino acid sequence, is formed very early. These stable structures that dominate the protein conformation form a framework, from which the protein assembles into its final form during folding of the polypeptide chains. The amino acid sequence of a protein contains all the information that determines its three-dimensional structure, and the structure of a protein determines the function thereof. Therefore, consistent with the genetic information, the nature of the functional information is that the stable structure determines the adaptive structure.

From the system perspective, the stability of a biological structural system is the self-stabilizing ability of an organic structural system formed by structural interaction. Each structure has structures that promote it and structures that inhibit it in the structural system, they form a dynamic balance in the interaction, that is, the structure is not lacked or excessive, and can be self-adjusted within a certain range according to environmental changes, so that the original ordered state is maintained and restored, namely, the order of the structure is maintained in an unbalanced state, wherein the interaction between the stable structures plays a dominant role. For example, in the recognition of structural information, the innate immune system in animals and plants can recognize the structural features of pathogens through pattern recognition receptors (PRRs). These features are stable molecular structures (e.g., cell walls composed of LPS in all bacteria) that are not easily mutated and are common to different types of pathogens. Take food allergies as an example, even if food containing allergens is steamed at high temperature, it will still cause allergies. The immune recognition recognizes the stable structure of allergens. Taking the neurotransmitter opioid peptide as another example, opioid peptides with different stereo conformations have different selectivity on cell membrane receptors, and endomorphin-1 is a biological active peptide with the highest affinity and selectivity on |_i-receptors known at present. The adaptive structure is the molecular basis for the binding of signal molecules to receptors, and in view of the change in its stable structure, amino acid, the difference between endomorphin-1 and other opioid peptides is mainly in three amino acids. Through the change of amino acid residues, the proper spatial layout is formed, and the spatial conformation is changed. This plays a role in correct positioning for this type of transmitter to bind to the receptor. The interaction of the stable structures can cause the obvious change of the adaptive structure, and plays a great role in information recognition. During the process of information transmission, receptors on cell membranes need to bring information into cell nuclei, which mainly depends on protein modification. The basis of protein modification is the chemical reactivity of amino acid residues in a primary structure. By adjusting the stable structure of the protein under the action of enzymes, the high order structure of the protein is more adapted to the functional requirements under specific time and space conditions. The modification has the characteristics of reversibility and versatility, such as phosphorylation and dephosphorylation of proteins. For the information transmitted to the nucleus, the cell will eventually make corresponding feedback through changes in gene expression, and continue to transmit instructions through the signal pathway. Therefore, the interaction or structural adjustment of the stable structure determines the change of the adaptive structure, which may, of course, also counteract the stable structure.

Among the biological structural systems, no structure is "isolated". The interaction, mutual restriction and interdependence between the structures and the sub-structure systems constitute the organic structural system of living beings. All organisms are composed of cells, and cells are the sub-structural systems of this large structural system of the body. Depending on the complexity of biological organisms, it is composed of hundreds, tens of thousands, or even hundreds of millions of cells that perform various specific functions. Various cells have different functions but cooperate with each other, thus forming an orderly and controllable cell society. The maintenance of this kind of sociality depends not only on the material metabolism and energy metabolism of the cell, but also on the communication and signal regulation between cells, so as to coordinate the behavior of the cell, such as cell growth, division, differentiation, apoptosis and other various physiological functions, and realize the complete life activity process of multicellular organisms. The essence of intercellular communication and signal regulation is also the interaction between structures, which determines the behavior and fate of cells, including structural and functional differentiation, location, survival, and death. The morphological structure, life activities and position of cells in the body are all regulated and controlled by the body, local tissues, surrounding cells and extracellular signal molecules, such as nerve cells, immune cells and endocrine cells, which participate in and maintain the homeostatic balance of the body through social connection. The interaction of structures in a biological structure system allows cells to have the ability to distinguish itself from foreign objects, and to distinguish between the same species and heterogeneous cells. The phenomenon of mutual recognition and identification between cells and the recognition of molecules of self and foreign substances is called cell recognition. Cells adhere to each other or establish a stable connection with certain morphological structure on the basis of recognition, thereby influencing and interacting with each other. Many vital activities are related to the recognition ability of cells. Cell recognition is also a very important link in the development and differentiation process of cells, which form different types of tissues through recognition and adhesion. Body immunity is actually a recognition phenomenon of cells. White blood cells in the blood can recognize invading bacteria and phagocytose them, but never phagocytose their own normal cells in the blood. This is the recognition of cells between different species. When the allogeneic tissue is transplanted clinically, the body will reject the transplanted allogeneic tissue, which is caused by the cell recognition between allogeneic cells. In addition, many physiological and pathological processes, such as blood coagulation, inflammatory reaction, thrombosis, infection by pathogenic microorganisms, and tumor cell metastasis, are related to the complex mutual recognition and adhesion between the same species or different species and the induced effects thereof. Structure recognition is based on interactions between structures that underlie the self-adaptive and self-organizing organic structural system formed by organisms.

The openness of the structural system is not only the openness of specific structures, but also the openness between relationships. The self-adaptive and self-organizing capabilities of the structural system are mainly reflected in the stability of the system under external environmental stimuli. The structural system of the body realizes self-adjustment and self-organization through the transmission and processing of information, and continuously overcomes the uncertainty of the structural system so as to keep the structural system in dynamic balance. The exchange between the structural system and its environment is not only the exchange of matter, but also the exchange of energy or information. To maintain homeostasis in a constantly changing environment, organisms need cells to have the ability to perceive these changes and respond. The ability of cells to receive and transmit information and execute instructions is the basis for organisms to respond to changes in internal and external environments. Proteins are the effectors of various physiological activities in cells (e.g., catalyzing chemical reactions and regulating gene expression), and changes in the state of the protein itself may also change its functional state. Organisms use protein molecules that are possessed by cells to build a signaling system. Because the shape of the protein molecule can be changed, the functions closely related to the shape of the protein molecule will change accordingly. The change in the state of a protein will change the state of downstream molecules by interacting with downstream protein molecules, and downstream molecules can change the state of further downstream molecules. Such successive changes of the state of each level are the way of information transmission in the cell. Furthermore, proteins are molecules with biological functions, which make the final response to signals after state changes and become effector molecules. The effector molecules at the end of the information transmission chain are mostly transcription factors, and respond to signals by regulating the expression of genes.

Taking the immune system as an example, any external structure entering the body will be recognized and evaluated by the immune system. For example, when bacteria or viruses enter the human body, immune cells interact with foreign structures to recognize their structural information, then secrete different cytokines according to the difference in structural information to transmit the information to B lymphocytes, regulate the gene expression of the B cells to synthesize IgG antibody, which binds to the antigen structure to form a complex and is eventually cleared by macrophages, so that the invasion of foreign matters is resisted. When parasitic pathogens invade, immune cells interact with foreign structures, and the recognized structural information activates B cells and regulates gene expression to synthesize IgE antibodies. These antibodies bind to mast cells and basophils, causing them to release toxic granular proteins and other substances, leading to the death of parasites.

During the immune response, the recognized structure determines the body's specific feedback. Through the interaction with the structure, the concrete structure is converted into the abstract structure, i.e., structural information. According to the recognition of the structural information, the body mobilizes multiple cells in the immune system to act synergistically, regulate gene expression, release different active substances to adapt to foreign objects, and realize a new stable and harmonious structural system. At the same time, the immune system interacts with other systems to exchange materials, energy, and information, so as to realize the coordination and cooperation between different subsystems in the structural system. It can be seen that there are structural information recognition and self-regulation in the structural system of the body, which restore the balance of the structural system, and even establish a new structural balance mechanism with the external structure.

A disease is an abnormal process of vital activities that occurs due to a disorder of homeostasis under certain etiology, and triggers a series of changes in metabolism, function, and structure, manifested as abnormal symptoms, signs and behaviors. The disease is an abnormal life process caused by self-stabilization regulation disorder after the organism is damaged under certain conditions. From a structural point of view, diseases of living beings are caused by imbalance in the structural systems of living beings, including: (1) structural imbalance within a biological structural system, (2) structural imbalance between a biological structural system and an external structure, i.e., the structural system of a living being lacks substances that inhibit the external structure. Therefore, substances related to diseases include: substance that causes the disease, substance of the organism itself and related to the disease, and substance that inhibits structure that causes the disease. The substance that inhibits the structure that causes the disease is a substance that can establish a structural balance with the structure that causes the disease. Disease is an extremely complex process. In many cases, from health to disease is a process from quantitative change to qualitative change. Some diseases involve the structural imbalance of multiple systems in the biological structure system. The "syndrome" of TCM reflects the essence of the disease, and the "syndrome" is differentiated and treated. The "syndrome" of TCM is neither a disease or a disorder as described by Western medicine, nor a generalization of a single factor or linear causal relationship of the disease, it refers to the understanding and generalization of the body's response state on various physiological and pathological associations such as the etiology, disease location, pathogenesis, pathological nature, and struggle between vital qi and pathogenic factors of a disease at certain stages. It focuses on the contradiction between vital qi and pathogenic factors of the human body. Therefore, strengthening the body resistance to eliminate pathogens and eliminating pathogens to calm the body resistance, which focus on both the body resistance and pathogens, regulate Yin and Yang of the human body, restore the relative balance of Yin and Yang, and coordinate the steady state of the body resistance and pathogens, are the basic rules of TCM.

Western medicine studies the relationship between the structure and the function from a concrete and micro perspective, thereby studying health and diseases; TCM studies the multi-dimensional and two-way regulation relationship of generation, restriction, control and transformation among human body functional systems from an abstract and macro perspective, thereby studying health and diseases. From a structural point of view, the essence of living things is a dynamic balanced, coordinated and orderly, self-adaptive, self-organizing organic structural system formed by the interaction between body structures. Structural information medicine integrates the basic theories of TCM and Western medicine with structure, recognizes life from the perspective of structural systems, and restores the dynamic balance of structural systems by allowing the body to recognize the information of specific structures and using the self-adaptation and self-organization functions of the body's structural system, so as to achieve the purpose of preventing and curing diseases.

Different lesions of the same organ can cause different diseases, and the symptoms of the same disease in different individuals are not exactly the same. In addition, the same disease may involve lesions of a plurality of different organs or tissues in a biological structural system, and the structures of the lesions of different individuals having the same disease are not identical. Therefore, the present invention combines the concrete structure of Western medicine research and the macroscopic theory established by TCM, treats and/or prevents diseases from the perspective of structural information. For the disease of the organism, only the substances related to the disease and the specific diseased cells, tissues or organs where the disease-related substances are located need to be known. There is no need to explore the mechanism through which the disease is caused, the specific symptoms of the disease, or the system where the disease-related substances are located. Whether in accordance with Western medicine or TCM, from a systematic point of view, people are the same, and even humans, pigs, cows, sheep, and primates are basically the same. Therefore, when the specific structure of the lesion cannot be determined or it is not easy to separate the structure of the lesion, treating and/or preventing the disease with the stable structure corresponding to the cells, tissues or organs containing the lesion structure (that is, the unbalanced structure) will be more versatile and easier to obtain and prepare. Therefore, the method for designing substances for the treatment and/or prevention of diseases disclosed in the present invention uses the stable structure corresponding to the substance related to diseases as an external structure to act on the structural system of the organism, and the structural system of the organism enable the organism to establish the balance with the structure of the external substance or restore the structural imbalance in the biological structural system according to structural self-adaptation and self-organization, thereby eliminating, relieving the diseases or preventing the diseases. For example, the stable structure corresponding to the lung of pig is used to treat and/or prevent asthma or allergic rhinitis, which is a disease on the nose that interacts with the structure of the lung, that is, a disease related to the lung. The method for designing substances for the treatment and/or prevention of diseases of the present invention mainly includes supporting vital qi with evil qi (pathogenic factor), and strengthening vital qi with vital qi.

The imbalance of the biological structure system includes the imbalance between the biological structure system and the structure other than itself. In the present invention, the biological structure system itself is called "vital qi", and the external structure that causes the imbalance of the biological structure system is called "external evil qi (exogenous pathogenic factor)". "External evil qi" is relative to living beings and includes substances that are harmful to healthy organisms, such as pathogenic bacteria, viruses, and the like, as well as substances that are not harmful to healthy organisms themselves, but for some organisms, may cause disease when these organisms come into contact with the substance. For example, a substance is harmless to a healthy living being, and even beneficial, i.e., the structure of the substance is balanced with the structure of the living being. However, if the body's structure is unbalanced, the balance between the structure of the substance and the structure of the organism is broken, diseases will occur, and different imbalances can cause different diseases, such as allergy, intolerance to the substance, such as allergy and intolerance to milk. Due to the functions of self-adaptation, self-organization, self-stabilization and the like of the biological structure system, under the action of the external structure, a structure that interacts with the external structure in an inhibitory or promoting manner will be produced, so that the balance between the biological structure system and the external structure is established. This is a way of supporting "vital qi" with "external evil qi".

The pathological changes of the internal structure of the biological structural system are often imbalance of structural interaction. For example, for a certain structure in the biological structural system, the lack of expression of genes of the structure that inhibits it or the overexpression of genes of the structure that promotes it, will break the balance between the structures. For example, tumor suppressor genes and proto-oncogenes restrict each other and maintain the relative stability of positive and negative regulatory signals. When such genes are mutated, deleted or inactivated, the balance of interactions between structures is broken, which can cause malignant transformation of cells and lead to tumors. In the present invention, the unbalanced structure in the biological structural system is called "internal evil qi (endogenous pathogenic factor)", and the biological structure system itself is called "vital qi". According to the present invention, the stable structure corresponding to the unbalanced substance inside the organism is used as an external structure to act on the biological structural system, and due to the functions of self-adaptation, self-organization, self-stabilization and the like of the biological structure system, the biological structure system can regulate the gene expression of the structure that interacts with the external structure according to the structural information in said external structure, so as to achieve a balance with the external structure, restore the balance of the unbalanced internal structure of the biological structure system, and cure diseases. This is a way of supporting "vital qi" with "internal evil qi".

Every normal structure in a biological structural system has a structure that interacts with it, i.e., a balanced structure, and the present invention refers to both the balanced structure and the biological structural system as "vital qi". The stable structure corresponding to the normal non-pathological substance in the system is used as an external structure to act on the biological structure system, and due to the functions of self-adaptation, self-organization, self-stabilization and the like of the biological structure system, the biological structure system can regulate the gene expression of the structure that interacts with the external structure to a normal level according to the structural information of each structure in said external structure, so the unbalanced structure naturally restores the balance. According to the present invention, the stable structure corresponding to the non-pathological substance is used as the substance for the treatment and/or prevention of diseases caused by the pathological changes of the same or similar substance as said non-pathological substance. This is a way of strengthening "vital qi" with "vital qi".

The balance of the biological structural system is dynamic, especially the balance between the biological structural system and the structure of the environment. For example, if a disease-causing substance does not cause disease in a certain organism, then the structural system of said organism and the structure of the disease-causing substance can reach a balance when interacting, in other words, in the structure of the organism related to the disease, there is a structure that interacts with the structure of the disease-causing substance to avoid the occurrence of the disease. Said structure does not exist in isolation in the biological structure system, and there are structures that interact with this structure in the biological system, thus forming a balance. The stable structure corresponding to the substance related to the disease in a certain organism is used as an external structure to act on a biological structure system in need of treatment said disease, and due to the functions of self-adaptation, self-organization, self-stabilization and the like of the biological structure system, the biological structure system can regulate the gene or gene expression of the structure that interacts with the external structure according to the structural information of each structure in the external structure, so as to achieve a balance between the biological structure system and the structure of the disease-causing substance. This is also is also a way of strengthening "vital qi" with "vital qi".

The invention is characterized in that:
The present invention is based on the established theoretical system of structural information medicine, uses the stable structure corresponding to the substance to act on the biological structural system, regulates the gene or the expression of the gene through the self-adapting, self-organizing and other abilities of the organism's own structural system, so as to treat the diseases related to the substance.

In view of this, the present invention provides substances for the treatment and/or prevention of allergic diseases, as well as design methods and preparation methods of said substances. The specific embodiments are as follows:
A method for designing substances for the treatment and/or prevention of allergic diseases, wherein the stable structure corresponding to the substance related to the allergic disease is used as the substance for treating and/or preventing the allergic disease.

Preferably, the stable structure corresponding to the substance refers to the structure of said substance that exists in a solid state at a high temperature of 500 °C or higher.

Preferably, the stable structure corresponding to the substance related to the allergic disease is selected from one or more of the following:
(1) the stable structure corresponding to the substance that causes allergic disease in the organism;
(2) the stable structure corresponding to the substance of the organism itself and related to allergic disease;
(3) the stable structure corresponding to the substance that inhibits the structure that causes allergic disease;
(4) the stable structure corresponding to the substance containing a substance of any one of (1) to (3) above.

The substance for treating and/or preventing allergic disease designed according to the above-mentioned method.

The preparation method of the above-mentioned substance for treating and/or preventing allergic disease, wherein the stable structure corresponding to the substance related to the allergic disease is prepared by a high-temperature carbonization method.

Use of the above-mentioned substance for treating and/or preventing allergic disease in the preparation of medicines, health products, foods, and food additives.

A method for designing substances for the treatment and/or prevention of food allergies, wherein the stable structure corresponding to the substance related to the food allergy is used as the substance for treating and/or preventing the food allergy.

Preferably, the stable structure corresponding to the substance refers to the structure of said substance that exists in a solid state at a high temperature of 500 °C or higher.

Preferably, the stable structure corresponding to the substance related to the food allergy is selected from one or more of the following:
(1) the stable structure corresponding to the substance that causes food allergy in the organism;
(2) the stable structure corresponding to the substance of the organism itself and related to food allergy;
(3) the stable structure corresponding to the substance that inhibits the structure that causes food allergy;
(4) the stable structure corresponding to the substance containing a substance of any one of (1) to (3) above.

The substance for treating and/or preventing food allergies designed according to the above-mentioned method.

The preparation method of the above-mentioned substance for treating and/or preventing food allergies, wherein the stable structure corresponding to the substance related to the food allergy is prepared by a high-temperature carbonization method.

Use of the above-mentioned substance for treating and/or preventing food allergies in the preparation of medicines, health products, foods, and food additives.

A method for designing substances for the treatment and/or prevention of peanut allergy, wherein the stable structure corresponding to the substance related to peanut allergy is used as the substance for treating and/or preventing peanut allergy.

Preferably, the stable structure corresponding to the substance refers to the structure of said substance that exists in a solid state at a high temperature of 500 °C or higher.

Preferably, the stable structure corresponding to the substance related to peanut allergy is selected from one or more of the following:
(1) the stable structure corresponding to the substance that causes peanut allergy in the organism;
(2) the stable structure corresponding to the substance of the organism itself and related to peanut allergy;
(3) the stable structure corresponding to the substance that inhibits the structure that causes peanut allergy;
(4) the stable structure corresponding to the substance containing a substance of any one of (1) to (3) above.

The substance for treating and/or preventing peanut allergy designed according to the above-mentioned method.

The preparation method of the above-mentioned substance for treating and/or preventing peanut allergy, wherein the stable structure corresponding to the substance related to peanut allergy is prepared by a high-temperature carbonization method.

Use of the above-mentioned substance for treating and/or preventing peanut allergy in the preparation of medicines, health products, foods, and food additives.

A method for designing substances for the treatment and/or prevention of egg allergy, wherein the stable structure corresponding to the substance related to egg allergy is used as the substance for treating and/or preventing egg allergy.

Preferably, the stable structure corresponding to the substance refers to the structure of said substance that exists in a solid state at a high temperature of 500 °C or higher.

Preferably, the stable structure corresponding to the substance related to egg allergy is selected from one or more of the following:
(1) the stable structure corresponding to the substance that causes egg allergy in the organism;
(2) the stable structure corresponding to the substance of the organism itself and related to egg allergy;
(3) the stable structure corresponding to the substance that inhibits the structure that causes egg allergy;
(4) the stable structure corresponding to the substance containing a substance of any one of (1) to (3) above.

The substance for treating and/or preventing egg allergy designed according to the above-mentioned method.

The preparation method of the above-mentioned substance for treating and/or preventing egg allergy, wherein the stable structure corresponding to the substance related to egg allergy is prepared by a high-temperature carbonization method.

Use of the above-mentioned substance for treating and/or preventing egg allergy in the preparation of medicines, health products, foods, and food additives.

A method for designing substances for the treatment and/or prevention of milk allergy, wherein the stable structure corresponding to the substance related to milk allergy is used as the substance for treating and/or preventing milk allergy.

Preferably, the stable structure corresponding to the substance refers to the structure of said substance that exists in a solid state at a high temperature of 500 °C or higher.

Preferably, the stable structure corresponding to the substance related to milk allergy is selected from one or more of the following:
(1) the stable structure corresponding to the substance that causes milk allergy in the organism;
(2) the stable structure corresponding to the substance of the organism itself and related to milk allergy;
(3) the stable structure corresponding to the substance that inhibits the structure that causes milk allergy;
(4) the stable structure corresponding to the substance containing a substance of any one of (1) to (3) above.

The substance for treating and/or preventing milk allergy designed according to the above-mentioned method.

The preparation method of the above-mentioned substance for treating and/or preventing milk allergy, wherein the stable structure corresponding to the substance related to milk allergy is prepared by a high-temperature carbonization method.

Use of the above-mentioned substance for treating and/or preventing milk allergy in the preparation of medicines, health products, foods, and food additives.

A method for designing substances for the treatment and/or prevention of soybean allergy, wherein the stable structure corresponding to the substance related to soybean allergy is used as the substance for treating and/or preventing soybean allergy.

Preferably, the stable structure corresponding to the substance refers to the structure of said substance that exists in a solid state at a high temperature of 500 °C or higher.

Preferably, the stable structure corresponding to the substance related to soybean allergy is selected from one or more of the following:
(1) the stable structure corresponding to the substance that causes soybean allergy in the organism;
(2) the stable structure corresponding to the substance of the organism itself and related to soybean allergy;
(3) the stable structure corresponding to the substance that inhibits the structure that causes soybean allergy;
(4) the stable structure corresponding to the substance containing a substance of any one of (1) to (3) above.

The substance for treating and/or preventing soybean allergy designed according to the above-mentioned method.

The preparation method of the above-mentioned substance for treating and/or preventing soybean allergy, wherein the stable structure corresponding to the substance related to soybean allergy is prepared by a high-temperature carbonization method.

Use of the above-mentioned substance for treating and/or preventing soybean allergy in the preparation of medicines, health products, foods, and food additives.

A method for designing substances for the treatment and/or prevention of nuts allergy, wherein the stable structure corresponding to the substance related to nuts allergy is used as the substance for treating and/or preventing nuts allergy.

Preferably, the stable structure corresponding to the substance refers to the structure of said substance that exists in a solid state at a high temperature of 500 °C or higher.

Preferably, the stable structure corresponding to the substance related to nuts allergy is selected from one or more of the following:
(1) the stable structure corresponding to the substance that causes nuts allergy in the organism;
(2) the stable structure corresponding to the substance of the organism itself and related to nuts allergy;
(3) the stable structure corresponding to the substance that inhibits the structure that causes nuts allergy;
(4) the stable structure corresponding to the substance containing a substance of any one of (1) to (3) above.

The substance for treating and/or preventing nuts allergy designed according to the above-mentioned method.

The preparation method of the above-mentioned substance for treating and/or preventing nuts allergy, wherein the stable structure corresponding to the substance related to nuts allergy is prepared by a high-temperature carbonization method.

Use of the above-mentioned substance for treating and/or preventing nuts allergy in the preparation of medicines, health products, foods, and food additives.

A method for designing substances for the treatment and/or prevention of fish allergy, wherein the stable structure corresponding to the substance related to fish allergy is used as the substance for treating and/or preventing fish allergy.

Preferably, the stable structure corresponding to the substance refers to the structure of said substance that exists in a solid state at a high temperature of 500 °C or higher.

Preferably, the stable structure corresponding to the substance related to fish allergy is selected from one or more of the following:
(1) the stable structure corresponding to the substance that causes fish allergy in the organism;
(2) the stable structure corresponding to the substance of the organism itself and related to fish allergy;
(3) the stable structure corresponding to the substance that inhibits the structure that causes fish allergy;
(4) the stable structure corresponding to the substance containing a substance of any one of (1) to (3) above.

The substance for treating and/or preventing fish allergy designed according to the above-mentioned method.

The preparation method of the above-mentioned substance for treating and/or preventing fish allergy, wherein the stable structure corresponding to the substance related to fish allergy is prepared by a high-temperature carbonization method.

Use of the above-mentioned substance for treating and/or preventing fish allergy in the preparation of medicines, health products, foods, and food additives.

A method for designing substances for the treatment and/or prevention of crustacean allergy, wherein the stable structure corresponding to the substance related to crustacean allergy is used as the substance for treating and/or preventing crustacean allergy.

Preferably, the stable structure corresponding to the substance refers to the structure of said substance that exists in a solid state at a high temperature of 500 °C or higher.

Preferably, the stable structure corresponding to the substance related to crustacean allergy is selected from one or more of the following:
(1) the stable structure corresponding to the substance that causes crustacean allergy in the organism;
(2) the stable structure corresponding to the substance of the organism itself and related to crustacean allergy;
(3) the stable structure corresponding to the substance that inhibits the structure that causes crustacean allergy;
(4) the stable structure corresponding to the substance containing a substance of any one of (1) to (3) above.

The substance for treating and/or preventing crustacean allergy designed according to the above-mentioned method.

The preparation method of the above-mentioned substance for treating and/or preventing crustacean allergy, wherein the stable structure corresponding to the substance related to crustacean allergy is prepared by a high-temperature carbonization method.

Use of the above-mentioned substance for treating and/or preventing crustacean allergy in the preparation of medicines, health products, foods, and food additives.

A method for designing substances for the treatment and/or prevention of wheat allergy, wherein the stable structure corresponding to the substance related to wheat allergy is used as the substance for treating and/or preventing wheat allergy.

Preferably, the stable structure corresponding to the substance refers to the structure of said substance that exists in a solid state at a high temperature of 500 °C or higher.

Preferably, the stable structure corresponding to the substance related to wheat allergy is selected from one or more of the following:
(1) the stable structure corresponding to the substance that causes wheat allergy in the organism;
(2) the stable structure corresponding to the substance of the organism itself and related to wheat allergy;
(3) the stable structure corresponding to the substance that inhibits the structure that causes wheat allergy;
(4) the stable structure corresponding to the substance containing a substance of any one of (1) to (3) above.

The substance for treating and/or preventing wheat allergy designed according to the above-mentioned method.

The preparation method of the above-mentioned substance for treating and/or preventing wheat allergy, wherein the stable structure corresponding to the substance related to wheat allergy is prepared by a high-temperature carbonization method.

Use of the above-mentioned substance for treating and/or preventing wheat allergy in the preparation of medicines, health products, foods, and food additives.

A method for designing substances for the treatment and/or prevention of shrimp allergy, wherein the stable structure corresponding to the substance related to shrimp allergy is used as the substance for treating and/or preventing shrimp allergy.

Preferably, the stable structure corresponding to the substance refers to the structure of said substance that exists in a solid state at a high temperature of 500 °C or higher.

Preferably, the stable structure corresponding to the substance related to shrimp allergy is selected from one or more of the following:
(1) the stable structure corresponding to the substance that causes shrimp allergy in the organism;
(2) the stable structure corresponding to the substance of the organism itself and related to shrimp allergy;
(3) the stable structure corresponding to the substance that inhibits the structure that causes shrimp allergy;
(4) the stable structure corresponding to the substance containing a substance of any one of (1) to (3) above.

The substance for treating and/or preventing shrimp allergy designed according to the above-mentioned method.

The preparation method of the above-mentioned substance for treating and/or preventing shrimp allergy, wherein the stable structure corresponding to the substance related to shrimp allergy is prepared by a high-temperature carbonization method.

Use of the above-mentioned substance for treating and/or preventing shrimp allergy in the preparation of medicines, health products, foods, and food additives.

A method for designing substances for the treatment and/or prevention of fruit allergy, wherein the stable structure corresponding to the substance related to fruit allergy is used as the substance for treating and/or preventing fruit allergy.

Preferably, the stable structure corresponding to the substance refers to the structure of said substance that exists in a solid state at a high temperature of 500 °C or higher.

Preferably, the stable structure corresponding to the substance related to fruit allergy is selected from one or more of the following:
(1) the stable structure corresponding to the substance that causes fruit allergy in the organism;
(2) the stable structure corresponding to the substance of the organism itself and related to fruit allergy;
(3) the stable structure corresponding to the substance that inhibits the structure that causes fruit allergy;
(4) the stable structure corresponding to the substance containing a substance of any one of (1) to (3) above.

The substance for treating and/or preventing fruit allergy designed according to the above-mentioned method.

The preparation method of the above-mentioned substance for treating and/or preventing fruit allergy, wherein the stable structure corresponding to the substance related to fruit allergy is prepared by a high-temperature carbonization method.

Use of the above-mentioned substance for treating and/or preventing fruit allergy in the preparation of medicines, health products, foods, and food additives.

A method for designing substances for the treatment and/or prevention of vegetable allergy, wherein the stable structure corresponding to the substance related to vegetable allergy is used as the substance for treating and/or preventing vegetable allergy.

Preferably, the stable structure corresponding to the substance refers to the structure of said substance that exists in a solid state at a high temperature of 500 °C or higher.

Preferably, the stable structure corresponding to the substance related to vegetable allergy is selected from one or more of the following:
(1) the stable structure corresponding to the substance that causes vegetable allergy in the organism;
(2) the stable structure corresponding to the substance of the organism itself and related to vegetable allergy;
(3) the stable structure corresponding to the substance that inhibits the structure that causes vegetable allergy;
(4) the stable structure corresponding to the substance containing a substance of any one of (1) to (3) above.

The substance for treating and/or preventing vegetable allergy designed according to the above-mentioned method.

The preparation method of the above-mentioned substance for treating and/or preventing vegetable allergy, wherein the stable structure corresponding to the substance related to vegetable allergy is prepared by a high-temperature carbonization method.

Use of the above-mentioned substance for treating and/or preventing vegetable allergy in the preparation of medicines, health products, foods, and food additives.

A method for designing substances for the treatment and/or prevention of plant seed allergy, wherein the stable structure corresponding to the substance related to plant seed allergy is used as the substance for treating and/or preventing plant seed allergy.

Preferably, the stable structure corresponding to the substance refers to the structure of said substance that exists in a solid state at a high temperature of 500 °C or higher.

Preferably, the stable structure corresponding to the substance related to plant seed allergy is selected from one or more of the following:
(1) the stable structure corresponding to the substance that causes plant seed allergy in the organism;
(2) the stable structure corresponding to the substance of the organism itself and related to plant seed allergy;
(3) the stable structure corresponding to the substance that inhibits the structure that causes plant seed allergy;
(4) the stable structure corresponding to the substance containing a substance of any one of (1) to (3) above.

The substance for treating and/or preventing plant seed allergy designed according to the above-mentioned method.

The preparation method of the above-mentioned substance for treating and/or preventing plant seed allergy, wherein the stable structure corresponding to the substance related to plant seed allergy is prepared by a high-temperature carbonization method.

Use of the above-mentioned substance for treating and/or preventing plant seed allergy in the preparation of medicines, health products, foods, and food additives.

A method for designing substances for the treatment and/or prevention of food additive allergy, wherein the stable structure corresponding to the substance related to food additive allergy is used as the substance for treating and/or preventing food additive allergy.

Preferably, the stable structure corresponding to the substance refers to the structure of said substance that exists in a solid state at a high temperature of 500 °C or higher.

Preferably, the stable structure corresponding to the substance related to food additive allergy is selected from one or more of the following:
(1) the stable structure corresponding to the substance that causes food additive allergy in the organism;
(2) the stable structure corresponding to the substance of the organism itself and related to food additive allergy;
(3) the stable structure corresponding to the substance that inhibits the structure that causes food additive allergy;
(4) the stable structure corresponding to the substance containing a substance of any one of (1) to (3) above.

The substance for treating and/or preventing food additive allergy designed according to the above-mentioned method.

The preparation method of the above-mentioned substance for treating and/or preventing food additive allergy, wherein the stable structure corresponding to the substance related to food additive allergy is prepared by a high-temperature carbonization method.

Use of the above-mentioned substance for treating and/or preventing food additive allergy in the preparation of medicines, health products, foods, and food additives.

A method for designing substances for the treatment and/or prevention of meat allergy, wherein the stable structure corresponding to the substance related to meat allergy is used as the substance for treating and/or preventing meat allergy.

Preferably, the stable structure corresponding to the substance refers to the structure of said substance that exists in a solid state at a high temperature of 500 °C or higher.

Preferably, the stable structure corresponding to the substance related to meat allergy is selected from one or more of the following:
(1) the stable structure corresponding to the substance that causes meat allergy in the organism;
(2) the stable structure corresponding to the substance of the organism itself and related to meat allergy;
(3) the stable structure corresponding to the substance that inhibits the structure that causes meat allergy;
(4) the stable structure corresponding to the substance containing a substance of any one of (1) to (3) above.

The substance for treating and/or preventing meat allergy designed according to the above-mentioned method.

The preparation method of the above-mentioned substance for treating and/or preventing meat allergy, wherein the stable structure corresponding to the substance related to meat allergy is prepared by a high-temperature carbonization method.

Use of the above-mentioned substance for treating and/or preventing meat allergy in the preparation of medicines, health products, foods, and food additives.

A method for designing substances for the treatment and/or prevention of room/house dust mite allergy, wherein the stable structure corresponding to the substance related to room/house dust mite allergy is used as the substance for treating and/or preventing room/house dust mite allergy.

Preferably, the stable structure corresponding to the substance refers to the structure of said substance that exists in a solid state at a high temperature of 500 °C or higher.

Preferably, the stable structure corresponding to the substance related to room/house dust mite allergy is selected from one or more of the following:
(1) the stable structure corresponding to the substance that causes room/house dust mite allergy in the organism;
(2) the stable structure corresponding to the substance of the organism itself and related to room/house dust mite allergy;
(3) the stable structure corresponding to the substance that inhibits the structure that causes room/house dust mite allergy;
(4) the stable structure corresponding to the substance containing a substance of any one of (1) to (3) above.

The substance for treating and/or preventing room/house dust mite allergy designed according to the above-mentioned method.

The preparation method of the above-mentioned substance for treating and/or preventing room/house dust mite allergy, wherein the stable structure corresponding to the substance related to room/house dust mite allergy is prepared by a high-temperature carbonization method.

Use of the above-mentioned substance for treating and/or preventing room/house dust mite allergy in the preparation of medicines, health products, foods, and food additives.

A method for designing substances for the treatment and/or prevention of dust mite allergy, wherein the stable structure corresponding to the substance related to dust mite allergy is used as the substance for treating and/or preventing dust mite allergy.

Preferably, the stable structure corresponding to the substance refers to the structure of said substance that exists in a solid state at a high temperature of 500 °C or higher.

Preferably, the stable structure corresponding to the substance related to dust mite allergy is selected from one or more of the following:
(1) the stable structure corresponding to the substance that causes dust mite allergy in the organism;
(2) the stable structure corresponding to the substance of the organism itself and related to dust mite allergy;
(3) the stable structure corresponding to the substance that inhibits the structure that causes dust mite allergy;
(4) the stable structure corresponding to the substance containing a substance of any one of (1) to (3) above.

The substance for treating and/or preventing dust mite allergy designed according to the above-mentioned method.

The preparation method of the above-mentioned substance for treating and/or preventing dust mite allergy, wherein the stable structure corresponding to the substance related to dust mite allergy is prepared by a high-temperature carbonization method.

Use of the above-mentioned substance for treating and/or preventing dust mite allergy in the preparation of medicines, health products, foods, and food additives.

A method for designing substances for the treatment and/or prevention of Alternaria allergy, wherein the stable structure corresponding to the substance related to Alternaria allergy is used as the substance for treating and/or preventing Alternaria allergy.

Preferably, the stable structure corresponding to the substance refers to the structure of said substance that exists in a solid state at a high temperature of 500 °C or higher.

Preferably, the stable structure corresponding to the substance related to Alternaria allergy is selected from one or more of the following:
(1) the stable structure corresponding to the substance that causes Alternaria allergy in the organism;
(2) the stable structure corresponding to the substance of the organism itself and related to Alternaria allergy;
(3) the stable structure corresponding to the substance that inhibits the structure that causes Alternaria allergy;
(4) the stable structure corresponding to the substance containing a substance of any one of (1) to (3) above.

The substance for treating and/or preventing Alternaria allergy designed according to the above-mentioned method.

The preparation method of the above-mentioned substance for treating and/or preventing Alternaria allergy, wherein the stable structure corresponding to the substance related to Alternaria allergy is prepared by a high-temperature carbonization method.

Use of the above-mentioned substance for treating and/or preventing Alternaria allergy in the preparation of medicines, health products, foods, and food additives.

A method for designing substances for the treatment and/or prevention of Aspergillus fumigatus allergy, wherein the stable structure corresponding to the substance related to Aspergillus fumigatus allergy is used as the substance for treating and/or preventing Aspergillus fumigatus allergy.

Preferably, the stable structure corresponding to the substance refers to the structure of said substance that exists in a solid state at a high temperature of 500 °C or higher.

Preferably, the stable structure corresponding to the substance related to Aspergillus fumigatus allergy is selected from one or more of the following:
(1) the stable structure corresponding to the substance that causes Aspergillus fumigatus allergy in the organism;
(2) the stable structure corresponding to the substance of the organism itself and related to Aspergillus fumigatus allergy;
(3) the stable structure corresponding to the substance that inhibits the structure that causes Aspergillus fumigatus allergy;
(4) the stable structure corresponding to the substance containing a substance of any one of (1) to (3) above.

The substance for treating and/or preventing Aspergillus fumigatus allergy designed according to the above-mentioned method.

The preparation method of the above-mentioned substance for treating and/or preventing Aspergillus fumigatus allergy, wherein the stable structure corresponding to the substance related to Aspergillus fumigatus allergy is prepared by a high-temperature carbonization method.

Use of the above-mentioned substance for treating and/or preventing Aspergillus fumigatus allergy in the preparation of medicines, health products, foods, and food additives.

A method for designing substances for the treatment and/or prevention of Candida albicans allergy, wherein the stable structure corresponding to the substance related to Candida albicans allergy is used as the substance for treating and/or preventing Candida albicans allergy.

Preferably, the stable structure corresponding to the substance refers to the structure of said substance that exists in a solid state at a high temperature of 500 °C or higher.

Preferably, the stable structure corresponding to the substance related to Candida albicans allergy is selected from one or more of the following:
(1) the stable structure corresponding to the substance that causes Candida albicans allergy in the organism;
(2) the stable structure corresponding to the substance of the organism itself and related to Candida albicans allergy;
(3) the stable structure corresponding to the substance that inhibits the structure that causes Candida albicans allergy;
(4) the stable structure corresponding to the substance containing a substance of any one of (1) to (3) above.

The substance for treating and/or preventing Candida albicans allergy designed according to the above-mentioned method.

The preparation method of the above-mentioned substance for treating and/or preventing Candida albicans allergy, wherein the stable structure corresponding to the substance related to Candida albicans allergy is prepared by a high-temperature carbonization method.

Use of the above-mentioned substance for treating and/or preventing Candida albicans allergy in the preparation of medicines, health products, foods, and food additives.

A method for designing substances for the treatment and/or prevention of Aspergillus niger allergy, wherein the stable structure corresponding to the substance related to Aspergillus niger allergy is used as the substance for treating and/or preventing Aspergillus niger allergy.

Preferably, the stable structure corresponding to the substance refers to the structure of said substance that exists in a solid state at a high temperature of 500 °C or higher.

Preferably, the stable structure corresponding to the substance related to Aspergillus niger allergy is selected from one or more of the following:
(1) the stable structure corresponding to the substance that causes Aspergillus niger allergy in the organism;
(2) the stable structure corresponding to the substance of the organism itself and related to Aspergillus niger allergy;
(3) the stable structure corresponding to the substance that inhibits the structure that causes Aspergillus niger allergy;
(4) the stable structure corresponding to the substance containing a substance of any one of (1) to (3) above.

The substance for treating and/or preventing Aspergillus niger allergy designed according to the above-mentioned method.

The preparation method of the above-mentioned substance for treating and/or preventing Aspergillus niger allergy, wherein the stable structure corresponding to the substance related to Aspergillus niger allergy is prepared by a high-temperature carbonization method.

Use of the above-mentioned substance for treating and/or preventing Aspergillus niger allergy in the preparation of medicines, health products, foods, and food additives.

A method for designing substances for the treatment and/or prevention of Cladosporium herbarum allergy, wherein the stable structure corresponding to the substance related to Cladosporium herbarum allergy is used as the substance for treating and/or preventing Cladosporium herbarum allergy.

Preferably, the stable structure corresponding to the substance refers to the structure of said substance that exists in a solid state at a high temperature of 500 °C or higher.

Preferably, the stable structure corresponding to the substance related to Cladosporium herbarum allergy is selected from one or more of the following:
(1) the stable structure corresponding to the substance that causes Cladosporium herbarum allergy in the organism;
(2) the stable structure corresponding to the substance of the organism itself and related to Cladosporium herbarum allergy;
(3) the stable structure corresponding to the substance that inhibits the structure that causes Cladosporium herbarum allergy;
(4) the stable structure corresponding to the substance containing a substance of any one of (1) to (3) above.

The substance for treating and/or preventing Cladosporium herbarum allergy designed according to the above-mentioned method.

The preparation method of the above-mentioned substance for treating and/or preventing Cladosporium herbarum allergy, wherein the stable structure corresponding to the substance related to Cladosporium herbarum allergy is prepared by a high-temperature carbonization method.

Use of the above-mentioned substance for treating and/or preventing Cladosporium herbarum allergy in the preparation of medicines, health products, foods, and food additives.

A method for designing substances for the treatment and/or prevention of dust allergy, wherein the stable structure corresponding to the substance related to dust allergy is used as the substance for treating and/or preventing dust allergy.

Preferably, the stable structure corresponding to the substance refers to the structure of said substance that exists in a solid state at a high temperature of 500 °C or higher.

Preferably, the stable structure corresponding to the substance related to dust allergy is selected from one or more of the following:
(1) the stable structure corresponding to the substance that causes dust allergy in the organism;
(2) the stable structure corresponding to the substance of the organism itself and related to dust allergy;
(3) the stable structure corresponding to the substance that inhibits the structure that causes dust allergy;
(4) the stable structure corresponding to the substance containing a substance of any one of (1) to (3) above.

The substance for treating and/or preventing dust allergy designed according to the above-mentioned method.

The preparation method of the above-mentioned substance for treating and/or preventing dust allergy, wherein the stable structure corresponding to the substance related to dust allergy is prepared by a high-temperature carbonization method.

Use of the above-mentioned substance for treating and/or preventing dust allergy in the preparation of medicines, health products, foods, and food additives.

A method for designing substances for the treatment and/or prevention of smoke allergy, wherein the stable structure corresponding to the substance related to smoke allergy is used as the substance for treating and/or preventing smoke allergy.

Preferably, the stable structure corresponding to the substance refers to the structure of said substance that exists in a solid state at a high temperature of 500 °C or higher.

Preferably, the stable structure corresponding to the substance related to smoke allergy is selected from one or more of the following:
(1) the stable structure corresponding to the substance that causes smoke allergy in the organism;
(2) the stable structure corresponding to the substance of the organism itself and related to smoke allergy;
(3) the stable structure corresponding to the substance that inhibits the structure that causes smoke allergy;
(4) the stable structure corresponding to the substance containing a substance of any one of (1) to (3) above.

The substance for treating and/or preventing smoke allergy designed according to the above-mentioned method.

The preparation method of the above-mentioned substance for treating and/or preventing smoke allergy, wherein the stable structure corresponding to the substance related to smoke allergy is prepared by a high-temperature carbonization method.

Use of the above-mentioned substance for treating and/or preventing smoke allergy in the preparation of medicines, health products, foods, and food additives.

A method for designing substances for the treatment and/or prevention of pollen allergy, wherein the stable structure corresponding to the substance related to pollen allergy is used as the substance for treating and/or preventing pollen allergy.

Preferably, the stable structure corresponding to the substance refers to the structure of said substance that exists in a solid state at a high temperature of 500 °C or higher.

Preferably, the stable structure corresponding to the substance related to pollen allergy is selected from one or more of the following:
(1) the stable structure corresponding to the substance that causes pollen allergy in the organism;
(2) the stable structure corresponding to the substance of the organism itself and related to pollen allergy;
(3) the stable structure corresponding to the substance that inhibits the structure that causes pollen allergy;
(4) the stable structure corresponding to the substance containing a substance of any one of (1) to (3) above.

The substance for treating and/or preventing pollen allergy designed according to the above-mentioned method.

The preparation method of the above-mentioned substance for treating and/or preventing pollen allergy, wherein the stable structure corresponding to the substance related to pollen allergy is prepared by a high-temperature carbonization method.

Use of the above-mentioned substance for treating and/or preventing pollen allergy in the preparation of medicines, health products, foods, and food additives.

A method for designing substances for the treatment and/or prevention of ragweed pollen allergy, wherein the stable structure corresponding to the substance related to ragweed pollen allergy is used as the substance for treating and/or preventing ragweed pollen allergy.

Preferably, the stable structure corresponding to the substance refers to the structure of said substance that exists in a solid state at a high temperature of 500 °C or higher.

Preferably, the stable structure corresponding to the substance related to ragweed pollen allergy is selected from one or more of the following:
(1) the stable structure corresponding to the substance that causes ragweed pollen allergy in the organism;
(2) the stable structure corresponding to the substance of the organism itself and related to ragweed pollen allergy;
(3) the stable structure corresponding to the substance that inhibits the structure that causes ragweed pollen allergy;
(4) the stable structure corresponding to the substance containing a substance of any one of (1) to (3) above.

The substance for treating and/or preventing ragweed pollen allergy designed according to the above-mentioned method.

The preparation method of the above-mentioned substance for treating and/or preventing ragweed pollen allergy, wherein the stable structure corresponding to the substance related to ragweed pollen allergy is prepared by a high-temperature carbonization method.

Use of the above-mentioned substance for treating and/or preventing ragweed pollen allergy in the preparation of medicines, health products, foods, and food additives.

A method for designing substances for the treatment and/or prevention of birch pollen allergy, wherein the stable structure corresponding to the substance related to birch pollen allergy is used as the substance for treating and/or preventing birch pollen allergy.

Preferably, the stable structure corresponding to the substance refers to the structure of said substance that exists in a solid state at a high temperature of 500 °C or higher.

Preferably, the stable structure corresponding to the substance related to birch pollen allergy is selected from one or more of the following:
(1) the stable structure corresponding to the substance that causes birch pollen allergy in the organism;
(2) the stable structure corresponding to the substance of the organism itself and related to birch pollen allergy;
(3) the stable structure corresponding to the substance that inhibits the structure that causes birch pollen allergy;
(4) the stable structure corresponding to the substance containing a substance of any one of (1) to (3) above.

The substance for treating and/or preventing birch pollen allergy designed according to the above-mentioned method.

The preparation method of the above-mentioned substance for treating and/or preventing birch pollen allergy, wherein the stable structure corresponding to the substance related to birch pollen allergy is prepared by a high-temperature carbonization method.

Use of the above-mentioned substance for treating and/or preventing birch pollen allergy in the preparation of medicines, health products, foods, and food additives.

A method for designing substances for the treatment and/or prevention of Artemisia pollen allergy, wherein the stable structure corresponding to the substance related to Artemisia pollen allergy is used as the substance for treating and/or preventing Artemisia pollen allergy.

Preferably, the stable structure corresponding to the substance refers to the structure of said substance that exists in a solid state at a high temperature of 500 °C or higher.

Preferably, the stable structure corresponding to the substance related to Artemisia pollen allergy is selected from one or more of the following:
(1) the stable structure corresponding to the substance that causes Artemisia pollen allergy in the organism;
(2) the stable structure corresponding to the substance of the organism itself and related to Artemisia pollen allergy;
(3) the stable structure corresponding to the substance that inhibits the structure that causes Artemisia pollen allergy;
(4) the stable structure corresponding to the substance containing a substance of any one of (1) to (3) above.

The substance for treating and/or preventing Artemisia pollen allergy designed according to the above-mentioned method.

The preparation method of the above-mentioned substance for treating and/or preventing Artemisia pollen allergy, wherein the stable structure corresponding to the substance related to Artemisia pollen allergy is prepared by a high-temperature carbonization method.

Use of the above-mentioned substance for treating and/or preventing Artemisia pollen allergy in the preparation of medicines, health products, foods, and food additives.

A method for designing substances for the treatment and/or prevention of grass pollen allergy, wherein the stable structure corresponding to the substance related to grass pollen allergy is used as the substance for treating and/or preventing grass pollen allergy.

Preferably, the stable structure corresponding to the substance refers to the structure of said substance that exists in a solid state at a high temperature of 500 °C or higher.

Preferably, the stable structure corresponding to the substance related to grass pollen allergy is selected from one or more of the following:
(1) the stable structure corresponding to the substance that causes grass pollen allergy in the organism;
(2) the stable structure corresponding to the substance of the organism itself and related to grass pollen allergy;
(3) the stable structure corresponding to the substance that inhibits the structure that causes grass pollen allergy;
(4) the stable structure corresponding to the substance containing a substance of any one of (1) to (3) above.

The substance for treating and/or preventing grass pollen allergy designed according to the above-mentioned method.

The preparation method of the above-mentioned substance for treating and/or preventing grass pollen allergy, wherein the stable structure corresponding to the substance related to grass pollen allergy is prepared by a high-temperature carbonization method.

Use of the above-mentioned substance for treating and/or preventing grass pollen allergy in the preparation of medicines, health products, foods, and food additives.

A method for designing substances for the treatment and/or prevention of cedar pollen allergy, wherein the stable structure corresponding to the substance related to cedar pollen allergy is used as the substance for treating and/or preventing cedar pollen allergy.

Preferably, the stable structure corresponding to the substance refers to the structure of said substance that exists in a solid state at a high temperature of 500 °C or higher.

Preferably, the stable structure corresponding to the substance related to cedar pollen allergy is selected from one or more of the following:
(1) the stable structure corresponding to the substance that causes cedar pollen allergy in the organism;
(2) the stable structure corresponding to the substance of the organism itself and related to cedar pollen allergy;
(3) the stable structure corresponding to the substance that inhibits the structure that causes cedar pollen allergy;
(4) the stable structure corresponding to the substance containing a substance of any one of (1) to (3) above.

The substance for treating and/or preventing cedar pollen allergy designed according to the above-mentioned method.

The preparation method of the above-mentioned substance for treating and/or preventing cedar pollen allergy, wherein the stable structure corresponding to the substance related to cedar pollen allergy is prepared by a high-temperature carbonization method.

Use of the above-mentioned substance for treating and/or preventing cedar pollen allergy in the preparation of medicines, health products, foods, and food additives.

A method for designing substances for the treatment and/or prevention of forage grass pollen allergy, wherein the stable structure corresponding to the substance related to forage grass pollen allergy is used as the substance for treating and/or preventing forage grass pollen allergy.

Preferably, the stable structure corresponding to the substance refers to the structure of said substance that exists in a solid state at a high temperature of 500 °C or higher.

Preferably, the stable structure corresponding to the substance related to forage grass pollen allergy is selected from one or more of the following:
(1) the stable structure corresponding to the substance that causes forage grass pollen allergy in the organism;
(2) the stable structure corresponding to the substance of the organism itself and related to forage grass pollen allergy;
(3) the stable structure corresponding to the substance that inhibits the structure that causes forage grass pollen allergy;
(4) the stable structure corresponding to the substance containing a substance of any one of (1) to (3) above.

The substance for treating and/or preventing forage grass pollen allergy designed according to the above-mentioned method.

The preparation method of the above-mentioned substance for treating and/or preventing forage grass pollen allergy, wherein the stable structure corresponding to the substance related to forage grass pollen allergy is prepared by a high-temperature carbonization method.

Use of the above-mentioned substance for treating and/or preventing forage grass pollen allergy in the preparation of medicines, health products, foods, and food additives.

A method for designing substances for the treatment and/or prevention of maple pollen allergy, wherein the stable structure corresponding to the substance related to maple pollen allergy is used as the substance for treating and/or preventing maple pollen allergy.

Preferably, the stable structure corresponding to the substance refers to the structure of said substance that exists in a solid state at a high temperature of 500 °C or higher.

Preferably, the stable structure corresponding to the substance related to maple pollen allergy is selected from one or more of the following:
(1) the stable structure corresponding to the substance that causes maple pollen allergy in the organism;
(2) the stable structure corresponding to the substance of the organism itself and related to maple pollen allergy;
(3) the stable structure corresponding to the substance that inhibits the structure that causes maple pollen allergy;
(4) the stable structure corresponding to the substance containing a substance of any one of (1) to (3) above.

The substance for treating and/or preventing maple pollen allergy designed according to the above-mentioned method.

The preparation method of the above-mentioned substance for treating and/or preventing maple pollen allergy, wherein the stable structure corresponding to the substance related to maple pollen allergy is prepared by a high-temperature carbonization method.

Use of the above-mentioned substance for treating and/or preventing maple pollen allergy in the preparation of medicines, health products, foods, and food additives.

A method for designing substances for the treatment and/or prevention of humulus pollen allergy, wherein the stable structure corresponding to the substance related to humulus pollen allergy is used as the substance for treating and/or preventing humulus pollen allergy.

Preferably, the stable structure corresponding to the substance refers to the structure of said substance that exists in a solid state at a high temperature of 500 °C or higher.

Preferably, the stable structure corresponding to the substance related to humulus pollen allergy is selected from one or more of the following:
(1) the stable structure corresponding to the substance that causes humulus pollen allergy in the organism;
(2) the stable structure corresponding to the substance of the organism itself and related to humulus pollen allergy;
(3) the stable structure corresponding to the substance that inhibits the structure that causes humulus pollen allergy;
(4) the stable structure corresponding to the substance containing a substance of any one of (1) to (3) above.

The substance for treating and/or preventing humulus pollen allergy designed according to the above-mentioned method.

The preparation method of the above-mentioned substance for treating and/or preventing humulus pollen allergy, wherein the stable structure corresponding to the substance related to humulus pollen allergy is prepared by a high-temperature carbonization method.

Use of the above-mentioned substance for treating and/or preventing humulus pollen allergy in the preparation of medicines, health products, foods, and food additives.

A method for designing substances for the treatment and/or prevention of white birch pollen allergy, wherein the stable structure corresponding to the substance related to white birch pollen allergy is used as the substance for treating and/or preventing white birch pollen allergy.

Preferably, the stable structure corresponding to the substance refers to the structure of said substance that exists in a solid state at a high temperature of 500 °C or higher.

Preferably, the stable structure corresponding to the substance related to white birch pollen allergy is selected from one or more of the following:
(1) the stable structure corresponding to the substance that causes white birch pollen allergy in the organism;
(2) the stable structure corresponding to the substance of the organism itself and related to white birch pollen allergy;
(3) the stable structure corresponding to the substance that inhibits the structure that causes white birch pollen allergy;
(4) the stable structure corresponding to the substance containing a substance of any one of (1) to (3) above.

The substance for treating and/or preventing white birch pollen allergy designed according to the above-mentioned method.

The preparation method of the above-mentioned substance for treating and/or preventing white birch pollen allergy, wherein the stable structure corresponding to the substance related to white birch pollen allergy is prepared by a high-temperature carbonization method.

Use of the above-mentioned substance for treating and/or preventing white birch pollen allergy in the preparation of medicines, health products, foods, and food additives.

A method for designing substances for the treatment and/or prevention of willow catkins allergy, wherein the stable structure corresponding to the substance related to willow catkins allergy is used as the substance for treating and/or preventing willow catkins allergy.

Preferably, the stable structure corresponding to the substance refers to the structure of said substance that exists in a solid state at a high temperature of 500 °C or higher.

Preferably, the stable structure corresponding to the substance related to willow catkins allergy is selected from one or more of the following:
(1) the stable structure corresponding to the substance that causes willow catkins allergy in the organism;
(2) the stable structure corresponding to the substance of the organism itself and related to willow catkins allergy;
(3) the stable structure corresponding to the substance that inhibits the structure that causes willow catkins allergy;
(4) the stable structure corresponding to the substance containing a substance of any one of (1) to (3) above.

The substance for treating and/or preventing willow catkins allergy designed according to the above-mentioned method.

The preparation method of the above-mentioned substance for treating and/or preventing willow catkins allergy, wherein the stable structure corresponding to the substance related to willow catkins allergy is prepared by a high-temperature carbonization method.

Use of the above-mentioned substance for treating and/or preventing willow catkins allergy in the preparation of medicines, health products, foods, and food additives.

A method for designing substances for the treatment and/or prevention of sycamore pollen allergy, wherein the stable structure corresponding to the substance related to sycamore pollen allergy is used as the substance for treating and/or preventing sycamore pollen allergy.

Preferably, the stable structure corresponding to the substance refers to the structure of said substance that exists in a solid state at a high temperature of 500 °C or higher.

Preferably, the stable structure corresponding to the substance related to sycamore pollen allergy is selected from one or more of the following:
(1) the stable structure corresponding to the substance that causes sycamore pollen allergy in the organism;
(2) the stable structure corresponding to the substance of the organism itself and related to sycamore pollen allergy;
(3) the stable structure corresponding to the substance that inhibits the structure that causes sycamore pollen allergy;
(4) the stable structure corresponding to the substance containing a substance of any one of (1) to (3) above.

The substance for treating and/or preventing sycamore pollen allergy designed according to the above-mentioned method.

The preparation method of the above-mentioned substance for treating and/or preventing sycamore pollen allergy, wherein the stable structure corresponding to the substance related to sycamore pollen allergy is prepared by a high-temperature carbonization method.

Use of the above-mentioned substance for treating and/or preventing sycamore pollen allergy in the preparation of medicines, health products, foods, and food additives.

A method for designing substances for the treatment and/or prevention of ginkgo pollen allergy, wherein the stable structure corresponding to the substance related to ginkgo pollen allergy is used as the substance for treating and/or preventing ginkgo pollen allergy.

Preferably, the stable structure corresponding to the substance refers to the structure of said substance that exists in a solid state at a high temperature of 500 °C or higher.

Preferably, the stable structure corresponding to the substance related to ginkgo pollen allergy is selected from one or more of the following:
(1) the stable structure corresponding to the substance that causes ginkgo pollen allergy in the organism;
(2) the stable structure corresponding to the substance of the organism itself and related to ginkgo pollen allergy;
(3) the stable structure corresponding to the substance that inhibits the structure that causes ginkgo pollen allergy;
(4) the stable structure corresponding to the substance containing a substance of any one of (1) to (3) above.

The substance for treating and/or preventing ginkgo pollen allergy designed according to the above-mentioned method.

The preparation method of the above-mentioned substance for treating and/or preventing ginkgo pollen allergy, wherein the stable structure corresponding to the substance related to ginkgo pollen allergy is prepared by a high-temperature carbonization method.

Use of the above-mentioned substance for treating and/or preventing ginkgo pollen allergy in the preparation of medicines, health products, foods, and food additives.

A method for designing substances for the treatment and/or prevention of cat hair allergy, wherein the stable structure corresponding to the substance related to cat hair allergy is used as the substance for treating and/or preventing cat hair allergy.

Preferably, the stable structure corresponding to the substance refers to the structure of said substance that exists in a solid state at a high temperature of 500 °C or higher.

Preferably, the stable structure corresponding to the substance related to cat hair allergy is selected from one or more of the following:
(1) the stable structure corresponding to the substance that causes cat hair allergy in the organism;
(2) the stable structure corresponding to the substance of the organism itself and related to cat hair allergy;
(3) the stable structure corresponding to the substance that inhibits the structure that causes cat hair allergy;
(4) the stable structure corresponding to the substance containing a substance of any one of (1) to (3) above.

The substance for treating and/or preventing cat hair allergy designed according to the above-mentioned method.

The preparation method of the above-mentioned substance for treating and/or preventing cat hair allergy, wherein the stable structure corresponding to the substance related to cat hair allergy is prepared by a high-temperature carbonization method.

Use of the above-mentioned substance for treating and/or preventing cat hair allergy in the preparation of medicines, health products, foods, and food additives.

A method for designing substances for the treatment and/or prevention of dog hair allergy, wherein the stable structure corresponding to the substance related to dog hair allergy is used as the substance for treating and/or preventing dog hair allergy.

Preferably, the stable structure corresponding to the substance refers to the structure of said substance that exists in a solid state at a high temperature of 500 °C or higher.

Preferably, the stable structure corresponding to the substance related to dog hair allergy is selected from one or more of the following:
(1) the stable structure corresponding to the substance that causes dog hair allergy in the organism;
(2) the stable structure corresponding to the substance of the organism itself and related to dog hair allergy;
(3) the stable structure corresponding to the substance that inhibits the structure that causes dog hair allergy;
(4) the stable structure corresponding to the substance containing a substance of any one of (1) to (3) above.

The substance for treating and/or preventing dog hair allergy designed according to the above-mentioned method.

The preparation method of the above-mentioned substance for treating and/or preventing dog hair allergy, wherein the stable structure corresponding to the substance related to dog hair allergy is prepared by a high-temperature carbonization method.

Use of the above-mentioned substance for treating and/or preventing dog hair allergy in the preparation of medicines, health products, foods, and food additives.

A method for designing substances for the treatment and/or prevention of cockroach allergy, wherein the stable structure corresponding to the substance related to cockroach allergy is used as the substance for treating and/or preventing cockroach allergy.

Preferably, the stable structure corresponding to the substance refers to the structure of said substance that exists in a solid state at a high temperature of 500 °C or higher.

Preferably, the stable structure corresponding to the substance related to cockroach allergy is selected from one or more of the following:
(1) the stable structure corresponding to the substance that causes cockroach allergy in the organism;
(2) the stable structure corresponding to the substance of the organism itself and related to cockroach allergy;
(3) the stable structure corresponding to the substance that inhibits the structure that causes cockroach allergy;
(4) the stable structure corresponding to the substance containing a substance of any one of (1) to (3) above.

The substance for treating and/or preventing cockroach allergy designed according to the above-mentioned method.

The preparation method of the above-mentioned substance for treating and/or preventing cockroach allergy, wherein the stable structure corresponding to the substance related to cockroach allergy is prepared by a high-temperature carbonization method.

Use of the above-mentioned substance for treating and/or preventing cockroach allergy in the preparation of medicines, health products, foods, and food additives.

A method for designing substances for the treatment and/or prevention of animal dander allergy, wherein the stable structure corresponding to the substance related to animal dander allergy is used as the substance for treating and/or preventing animal dander allergy.

Preferably, the stable structure corresponding to the substance refers to the structure of said substance that exists in a solid state at a high temperature of 500 °C or higher.

Preferably, the stable structure corresponding to the substance related to animal dander allergy is selected from one or more of the following:
(1) the stable structure corresponding to the substance that causes animal dander allergy in the organism;
(2) the stable structure corresponding to the substance of the organism itself and related to animal dander allergy;
(3) the stable structure corresponding to the substance that inhibits the structure that causes animal dander allergy;
(4) the stable structure corresponding to the substance containing a substance of any one of (1) to (3) above.

The substance for treating and/or preventing animal dander allergy designed according to the above-mentioned method.

The preparation method of the above-mentioned substance for treating and/or preventing animal dander allergy, wherein the stable structure corresponding to the substance related to animal dander allergy is prepared by a high-temperature carbonization method.

Use of the above-mentioned substance for treating and/or preventing animal dander allergy in the preparation of medicines, health products, foods, and food additives.

A method for designing substances for the treatment and/or prevention of insect secretions allergy, wherein the stable structure corresponding to the substance related to insect secretions allergy is used as the substance for treating and/or preventing insect secretions allergy.

Preferably, the stable structure corresponding to the substance refers to the structure of said substance that exists in a solid state at a high temperature of 500 °C or higher.

Preferably, the stable structure corresponding to the substance related to insect secretions allergy is selected from one or more of the following:
(1) the stable structure corresponding to the substance that causes insect secretions allergy in the organism;
(2) the stable structure corresponding to the substance of the organism itself and related to insect secretions allergy;
(3) the stable structure corresponding to the substance that inhibits the structure that causes insect secretions allergy;
(4) the stable structure corresponding to the substance containing a substance of any one of (1) to (3) above.

The substance for treating and/or preventing insect secretions allergy designed according to the above-mentioned method.

The preparation method of the above-mentioned substance for treating and/or preventing insect secretions allergy, wherein the stable structure corresponding to the substance related to insect secretions allergy is prepared by a high-temperature carbonization method.

Use of the above-mentioned substance for treating and/or preventing insect secretions allergy in the preparation of medicines, health products, foods, and food additives.

A method for designing substances for the treatment and/or prevention of feather allergy, wherein the stable structure corresponding to the substance related to feather allergy is used as the substance for treating and/or preventing feather allergy.

Preferably, the stable structure corresponding to the substance refers to the structure of said substance that exists in a solid state at a high temperature of 500 °C or higher.

Preferably, the stable structure corresponding to the substance related to feather allergy is selected from one or more of the following:
(1) the stable structure corresponding to the substance that causes feather allergy in the organism;
(2) the stable structure corresponding to the substance of the organism itself and related to feather allergy;
(3) the stable structure corresponding to the substance that inhibits the structure that causes feather allergy;
(4) the stable structure corresponding to the substance containing a substance of any one of (1) to (3) above.

The substance for treating and/or preventing feather allergy designed according to the above-mentioned method.

The preparation method of the above-mentioned substance for treating and/or preventing feather allergy, wherein the stable structure corresponding to the substance related to feather allergy is prepared by a high-temperature carbonization method.

Use of the above-mentioned substance for treating and/or preventing feather allergy in the preparation of medicines, health products, foods, and food additives.

A method for designing substances for the treatment and/or prevention of pigeon droppings allergy, wherein the stable structure corresponding to the substance related to pigeon droppings allergy is used as the substance for treating and/or preventing pigeon droppings allergy.

Preferably, the stable structure corresponding to the substance refers to the structure of said substance that exists in a solid state at a high temperature of 500 °C or higher.

Preferably, the stable structure corresponding to the substance related to pigeon droppings allergy is selected from one or more of the following:
(1) the stable structure corresponding to the substance that causes pigeon droppings allergy in the organism;
(2) the stable structure corresponding to the substance of the organism itself and related to pigeon droppings allergy;
(3) the stable structure corresponding to the substance that inhibits the structure that causes pigeon droppings allergy;
(4) the stable structure corresponding to the substance containing a substance of any one of (1) to (3) above.

The substance for treating and/or preventing pigeon droppings allergy designed according to the above-mentioned method.

The preparation method of the above-mentioned substance for treating and/or preventing pigeon droppings allergy, wherein the stable structure corresponding to the substance related to pigeon droppings allergy is prepared by a high-temperature carbonization method.

Use of the above-mentioned substance for treating and/or preventing pigeon droppings allergy in the preparation of medicines, health products, foods, and food additives.

A method for designing substances for the treatment and/or prevention of budgie allergy, wherein the stable structure corresponding to the substance related to budgie allergy is used as the substance for treating and/or preventing budgie allergy.

Preferably, the stable structure corresponding to the substance refers to the structure of said substance that exists in a solid state at a high temperature of 500 °C or higher.

Preferably, the stable structure corresponding to the substance related to budgie allergy is selected from one or more of the following:
(1) the stable structure corresponding to the substance that causes budgie allergy in the organism;
(2) the stable structure corresponding to the substance of the organism itself and related to budgie allergy;
(3) the stable structure corresponding to the substance that inhibits the structure that causes budgie allergy;
(4) the stable structure corresponding to the substance containing a substance of any one of (1) to (3) above.

The substance for treating and/or preventing budgie allergy designed according to the above-mentioned method.

The preparation method of the above-mentioned substance for treating and/or preventing budgie allergy, wherein the stable structure corresponding to the substance related to budgie allergy is prepared by a high-temperature carbonization method.

Use of the above-mentioned substance for treating and/or preventing budgie allergy in the preparation of medicines, health products, foods, and food additives.

A method for designing substances for the treatment and/or prevention of drug allergy, wherein the stable structure corresponding to the substance related to drug allergy is used as the substance for treating and/or preventing drug allergy.

Preferably, the stable structure corresponding to the substance refers to the structure of said substance that exists in a solid state at a high temperature of 500 °C or higher.

Preferably, the stable structure corresponding to the substance related to drug allergy is selected from one or more of the following:
(1) the stable structure corresponding to the substance that causes drug allergy in the organism;
(2) the stable structure corresponding to the substance of the organism itself and related to drug allergy;
(3) the stable structure corresponding to the substance that inhibits the structure that causes drug allergy;
(4) the stable structure corresponding to the substance containing a substance of any one of (1) to (3) above.

The substance for treating and/or preventing drug allergy designed according to the above-mentioned method.

The preparation method of the above-mentioned substance for treating and/or preventing drug allergy, wherein the stable structure corresponding to the substance related to drug allergy is prepared by a high-temperature carbonization method.

Use of the above-mentioned substance for treating and/or preventing drug allergy in the preparation of medicines, health products, foods, and food additives.

A method for designing substances for the treatment and/or prevention of penicillin allergy, wherein the stable structure corresponding to the substance related to penicillin allergy is used as the substance for treating and/or preventing penicillin allergy.

Preferably, the stable structure corresponding to the substance refers to the structure of said substance that exists in a solid state at a high temperature of 500 °C or higher.

Preferably, the stable structure corresponding to the substance related to penicillin allergy is selected from one or more of the following:
(1) the stable structure corresponding to the substance that causes penicillin allergy in the organism;
(2) the stable structure corresponding to the substance of the organism itself and related to penicillin allergy;
(3) the stable structure corresponding to the substance that inhibits the structure that causes penicillin allergy;
(4) the stable structure corresponding to the substance containing a substance of any one of (1) to (3) above.

The substance for treating and/or preventing penicillin allergy designed according to the above-mentioned method.

The preparation method of the above-mentioned substance for treating and/or preventing penicillin allergy, wherein the stable structure corresponding to the substance related to penicillin allergy is prepared by a high-temperature carbonization method.

Use of the above-mentioned substance for treating and/or preventing penicillin allergy in the preparation of medicines, health products, foods, and food additives.

A method for designing substances for the treatment and/or prevention of azithromycin allergy, wherein the stable structure corresponding to the substance related to azithromycin allergy is used as the substance for treating and/or preventing azithromycin allergy.

Preferably, the stable structure corresponding to the substance refers to the structure of said substance that exists in a solid state at a high temperature of 500 °C or higher.

Preferably, the stable structure corresponding to the substance related to azithromycin allergy is selected from one or more of the following:
(1) the stable structure corresponding to the substance that causes azithromycin allergy in the organism;
(2) the stable structure corresponding to the substance of the organism itself and related to azithromycin allergy;
(3) the stable structure corresponding to the substance that inhibits the structure that causes azithromycin allergy;
(4) the stable structure corresponding to the substance containing a substance of any one of (1) to (3) above.

The substance for treating and/or preventing azithromycin allergy designed according to the above-mentioned method.

The preparation method of the above-mentioned substance for treating and/or preventing azithromycin allergy, wherein the stable structure corresponding to the substance related to azithromycin allergy is prepared by a high-temperature carbonization method.

Use of the above-mentioned substance for treating and/or preventing azithromycin allergy in the preparation of medicines, health products, foods, and food additives.

A method for designing substances for the treatment and/or prevention of cephalosporins allergy, wherein the stable structure corresponding to the substance related to cephalosporins allergy is used as the substance for treating and/or preventing cephalosporins allergy.

Preferably, the stable structure corresponding to the substance refers to the structure of said substance that exists in a solid state at a high temperature of 500 °C or higher.

Preferably, the stable structure corresponding to the substance related to cephalosporins allergy is selected from one or more of the following:
(1) the stable structure corresponding to the substance that causes cephalosporins allergy in the organism;
(2) the stable structure corresponding to the substance of the organism itself and related to cephalosporins allergy;
(3) the stable structure corresponding to the substance that inhibits the structure that causes cephalosporins allergy;
(4) the stable structure corresponding to the substance containing a substance of any one of (1) to (3) above.

The substance for treating and/or preventing cephalosporins allergy designed according to the above-mentioned method.

The preparation method of the above-mentioned substance for treating and/or preventing cephalosporins allergy, wherein the stable structure corresponding to the substance related to cephalosporins allergy is prepared by a high-temperature carbonization method.

Use of the above-mentioned substance for treating and/or preventing cephalosporins allergy in the preparation of medicines, health products, foods, and food additives.

A method for designing substances for the treatment and/or prevention of erythromycin allergy, wherein the stable structure corresponding to the substance related to erythromycin allergy is used as the substance for treating and/or preventing erythromycin allergy.

Preferably, the stable structure corresponding to the substance refers to the structure of said substance that exists in a solid state at a high temperature of 500 °C or higher.

Preferably, the stable structure corresponding to the substance related to erythromycin allergy is selected from one or more of the following:
(1) the stable structure corresponding to the substance that causes erythromycin allergy in the organism;
(2) the stable structure corresponding to the substance of the organism itself and related to erythromycin allergy;
(3) the stable structure corresponding to the substance that inhibits the structure that causes erythromycin allergy;
(4) the stable structure corresponding to the substance containing a substance of any one of (1) to (3) above.

The substance for treating and/or preventing erythromycin allergy designed according to the above-mentioned method.

The preparation method of the above-mentioned substance for treating and/or preventing erythromycin allergy, wherein the stable structure corresponding to the substance related to erythromycin allergy is prepared by a high-temperature carbonization method.

Use of the above-mentioned substance for treating and/or preventing erythromycin allergy in the preparation of medicines, health products, foods, and food additives.

A method for designing substances for the treatment and/or prevention of chloramphenicol allergy, wherein the stable structure corresponding to the substance related to chloramphenicol allergy is used as the substance for treating and/or preventing chloramphenicol allergy.

Preferably, the stable structure corresponding to the substance refers to the structure of said substance that exists in a solid state at a high temperature of 500 °C or higher.

Preferably, the stable structure corresponding to the substance related to chloramphenicol allergy is selected from one or more of the following:
(1) the stable structure corresponding to the substance that causes chloramphenicol allergy in the organism;
(2) the stable structure corresponding to the substance of the organism itself and related to chloramphenicol allergy;
(3) the stable structure corresponding to the substance that inhibits the structure that causes chloramphenicol allergy;
(4) the stable structure corresponding to the substance containing a substance of any one of (1) to (3) above.

The substance for treating and/or preventing chloramphenicol allergy designed according to the above-mentioned method.

The preparation method of the above-mentioned substance for treating and/or preventing chloramphenicol allergy, wherein the stable structure corresponding to the substance related to chloramphenicol allergy is prepared by a high-temperature carbonization method.

Use of the above-mentioned substance for treating and/or preventing chloramphenicol allergy in the preparation of medicines, health products, foods, and food additives.

A method for designing substances for the treatment and/or prevention of levofloxacin allergy, wherein the stable structure corresponding to the substance related to levofloxacin allergy is used as the substance for treating and/or preventing levofloxacin allergy.

Preferably, the stable structure corresponding to the substance refers to the structure of said substance that exists in a solid state at a high temperature of 500 °C or higher.

Preferably, the stable structure corresponding to the substance related to levofloxacin allergy is selected from one or more of the following:
(1) the stable structure corresponding to the substance that causes levofloxacin allergy in the organism;
(2) the stable structure corresponding to the substance of the organism itself and related to levofloxacin allergy;
(3) the stable structure corresponding to the substance that inhibits the structure that causes levofloxacin allergy;
(4) the stable structure corresponding to the substance containing a substance of any one of (1) to (3) above.

The substance for treating and/or preventing levofloxacin allergy designed according to the above-mentioned method.

The preparation method of the above-mentioned substance for treating and/or preventing levofloxacin allergy, wherein the stable structure corresponding to the substance related to levofloxacin allergy is prepared by a high-temperature carbonization method.

Use of the above-mentioned substance for treating and/or preventing levofloxacin allergy in the preparation of medicines, health products, foods, and food additives.

A method for designing substances for the treatment and/or prevention of amoxicillin allergy, wherein the stable structure corresponding to the substance related to amoxicillin allergy is used as the substance for treating and/or preventing amoxicillin allergy.

Preferably, the stable structure corresponding to the substance refers to the structure of said substance that exists in a solid state at a high temperature of 500 °C or higher.

Preferably, the stable structure corresponding to the substance related to amoxicillin allergy is selected from one or more of the following:
(1) the stable structure corresponding to the substance that causes amoxicillin allergy in the organism;
(2) the stable structure corresponding to the substance of the organism itself and related to amoxicillin allergy;
(3) the stable structure corresponding to the substance that inhibits the structure that causes amoxicillin allergy;
(4) the stable structure corresponding to the substance containing a substance of any one of (1) to (3) above.

The substance for treating and/or preventing amoxicillin allergy designed according to the above-mentioned method.

The preparation method of the above-mentioned substance for treating and/or preventing amoxicillin allergy, wherein the stable structure corresponding to the substance related to amoxicillin allergy is prepared by a high-temperature carbonization method.

Use of the above-mentioned substance for treating and/or preventing amoxicillin allergy in the preparation of medicines, health products, foods, and food additives.

A method for designing substances for the treatment and/or prevention of cephalosporin allergy, wherein the stable structure corresponding to the substance related to cephalosporin allergy is used as the substance for treating and/or preventing cephalosporin allergy.

Preferably, the stable structure corresponding to the substance refers to the structure of said substance that exists in a solid state at a high temperature of 500 °C or higher.

Preferably, the stable structure corresponding to the substance related to cephalosporin allergy is selected from one or more of the following:
(1) the stable structure corresponding to the substance that causes cephalosporin allergy in the organism;
(2) the stable structure corresponding to the substance of the organism itself and related to cephalosporin allergy;
(3) the stable structure corresponding to the substance that inhibits the structure that causes cephalosporin allergy;
(4) the stable structure corresponding to the substance containing a substance of any one of (1) to (3) above.

The substance for treating and/or preventing cephalosporin allergy designed according to the above-mentioned method.

The preparation method of the above-mentioned substance for treating and/or preventing cephalosporin allergy, wherein the stable structure corresponding to the substance related to cephalosporin allergy is prepared by a high-temperature carbonization method.

Use of the above-mentioned substance for treating and/or preventing cephalosporin allergy in the preparation of medicines, health products, foods, and food additives.

A method for designing substances for the treatment and/or prevention of neomycin sulfate allergy, wherein the stable structure corresponding to the substance related to neomycin sulfate allergy is used as the substance for treating and/or preventing neomycin sulfate allergy.

Preferably, the stable structure corresponding to the substance refers to the structure of said substance that exists in a solid state at a high temperature of 500 °C or higher.

Preferably, the stable structure corresponding to the substance related to neomycin sulfate allergy is selected from one or more of the following:
(1) the stable structure corresponding to the substance that causes neomycin sulfate allergy in the organism;
(2) the stable structure corresponding to the substance of the organism itself and related to neomycin sulfate allergy;
(3) the stable structure corresponding to the substance that inhibits the structure that causes neomycin sulfate allergy;
(4) the stable structure corresponding to the substance containing a substance of any one of (1) to (3) above.

The substance for treating and/or preventing neomycin sulfate allergy designed according to the above-mentioned method.

The preparation method of the above-mentioned substance for treating and/or preventing neomycin sulfate allergy, wherein the stable structure corresponding to the substance related to neomycin sulfate allergy is prepared by a high-temperature carbonization method.

Use of the above-mentioned substance for treating and/or preventing neomycin sulfate allergy in the preparation of medicines, health products, foods, and food additives.

A method for designing substances for the treatment and/or prevention of tetracycline allergy, wherein the stable structure corresponding to the substance related to tetracycline allergy is used as the substance for treating and/or preventing tetracycline allergy.

Preferably, the stable structure corresponding to the substance refers to the structure of said substance that exists in a solid state at a high temperature of 500 °C or higher.

Preferably, the stable structure corresponding to the substance related to tetracycline allergy is selected from one or more of the following:
(1) the stable structure corresponding to the substance that causes tetracycline allergy in the organism;
(2) the stable structure corresponding to the substance of the organism itself and related to tetracycline allergy;
(3) the stable structure corresponding to the substance that inhibits the structure that causes tetracycline allergy;
(4) the stable structure corresponding to the substance containing a substance of any one of (1) to (3) above.

The substance for treating and/or preventing tetracycline allergy designed according to the above-mentioned method.

The preparation method of the above-mentioned substance for treating and/or preventing tetracycline allergy, wherein the stable structure corresponding to the substance related to tetracycline allergy is prepared by a high-temperature carbonization method.

Use of the above-mentioned substance for treating and/or preventing tetracycline allergy in the preparation of medicines, health products, foods, and food additives.

A method for designing substances for the treatment and/or prevention of anesthetic allergy, wherein the stable structure corresponding to the substance related to anesthetic allergy is used as the substance for treating and/or preventing anesthetic allergy.

Preferably, the stable structure corresponding to the substance refers to the structure of said substance that exists in a solid state at a high temperature of 500 °C or higher.

Preferably, the stable structure corresponding to the substance related to anesthetic allergy is selected from one or more of the following:
(1) the stable structure corresponding to the substance that causes anesthetic allergy in the organism;
(2) the stable structure corresponding to the substance of the organism itself and related to anesthetic allergy;
(3) the stable structure corresponding to the substance that inhibits the structure that causes anesthetic allergy;
(4) the stable structure corresponding to the substance containing a substance of any one of (1) to (3) above.

The substance for treating and/or preventing anesthetic allergy designed according to the above-mentioned method.

The preparation method of the above-mentioned substance for treating and/or preventing anesthetic allergy, wherein the stable structure corresponding to the substance related to anesthetic allergy is prepared by a high-temperature carbonization method.

Use of the above-mentioned substance for treating and/or preventing anesthetic allergy in the preparation of medicines, health products, foods, and food additives.

A method for designing substances for the treatment and/or prevention of codeine allergy, wherein the stable structure corresponding to the substance related to codeine allergy is used as the substance for treating and/or preventing codeine allergy.

Preferably, the stable structure corresponding to the substance refers to the structure of said substance that exists in a solid state at a high temperature of 500 °C or higher.

Preferably, the stable structure corresponding to the substance related to codeine allergy is selected from one or more of the following:
(1) the stable structure corresponding to the substance that causes codeine allergy in the organism;
(2) the stable structure corresponding to the substance of the organism itself and related to codeine allergy;
(3) the stable structure corresponding to the substance that inhibits the structure that causes codeine allergy;
(4) the stable structure corresponding to the substance containing a substance of any one of (1) to (3) above.

The substance for treating and/or preventing codeine allergy designed according to the above-mentioned method.

The preparation method of the above-mentioned substance for treating and/or preventing codeine allergy, wherein the stable structure corresponding to the substance related to codeine allergy is prepared by a high-temperature carbonization method.

Use of the above-mentioned substance for treating and/or preventing codeine allergy in the preparation of medicines, health products, foods, and food additives.

A method for designing substances for the treatment and/or prevention of dilantin allergy, wherein the stable structure corresponding to the substance related to dilantin allergy is used as the substance for treating and/or preventing dilantin allergy.

Preferably, the stable structure corresponding to the substance refers to the structure of said substance that exists in a solid state at a high temperature of 500 °C or higher.

Preferably, the stable structure corresponding to the substance related to dilantin allergy is selected from one or more of the following:
(1) the stable structure corresponding to the substance that causes dilantin allergy in the organism;
(2) the stable structure corresponding to the substance of the organism itself and related to dilantin allergy;
(3) the stable structure corresponding to the substance that inhibits the structure that causes dilantin allergy;
(4) the stable structure corresponding to the substance containing a substance of any one of (1) to (3) above.

The substance for treating and/or preventing dilantin allergy designed according to the above-mentioned method.

The preparation method of the above-mentioned substance for treating and/or preventing dilantin allergy, wherein the stable structure corresponding to the substance related to dilantin allergy is prepared by a high-temperature carbonization method.

Use of the above-mentioned substance for treating and/or preventing dilantin allergy in the preparation of medicines, health products, foods, and food additives.

A method for designing substances for the treatment and/or prevention of carbamazepine allergy, wherein the stable structure corresponding to the substance related to carbamazepine allergy is used as the substance for treating and/or preventing carbamazepine allergy.

Preferably, the stable structure corresponding to the substance refers to the structure of said substance that exists in a solid state at a high temperature of 500 °C or higher.

Preferably, the stable structure corresponding to the substance related to carbamazepine allergy is selected from one or more of the following:
(1) the stable structure corresponding to the substance that causes carbamazepine allergy in the organism;
(2) the stable structure corresponding to the substance of the organism itself and related to carbamazepine allergy;
(3) the stable structure corresponding to the substance that inhibits the structure that causes carbamazepine allergy;
(4) the stable structure corresponding to the substance containing a substance of any one of (1) to (3) above.

The substance for treating and/or preventing carbamazepine allergy designed according to the above-mentioned method.

The preparation method of the above-mentioned substance for treating and/or preventing carbamazepine allergy, wherein the stable structure corresponding to the substance related to carbamazepine allergy is prepared by a high-temperature carbonization method.

Use of the above-mentioned substance for treating and/or preventing carbamazepine allergy in the preparation of medicines, health products, foods, and food additives.

A method for designing substances for the treatment and/or prevention of sulfonamides allergy, wherein the stable structure corresponding to the substance related to sulfonamides allergy is used as the substance for treating and/or preventing sulfonamides allergy.

Preferably, the stable structure corresponding to the substance refers to the structure of said substance that exists in a solid state at a high temperature of 500 °C or higher.

Preferably, the stable structure corresponding to the substance related to sulfonamides allergy is selected from one or more of the following:
(1) the stable structure corresponding to the substance that causes sulfonamides allergy in the organism;
(2) the stable structure corresponding to the substance of the organism itself and related to sulfonamides allergy;
(3) the stable structure corresponding to the substance that inhibits the structure that causes sulfonamides allergy;
(4) the stable structure corresponding to the substance containing a substance of any one of (1) to (3) above.

The substance for treating and/or preventing sulfonamides allergy designed according to the above-mentioned method.

The preparation method of the above-mentioned substance for treating and/or preventing sulfonamides allergy, wherein the stable structure corresponding to the substance related to sulfonamides allergy is prepared by a high-temperature carbonization method.

Use of the above-mentioned substance for treating and/or preventing sulfonamides allergy in the preparation of medicines, health products, foods, and food additives.

A method for designing substances for the treatment and/or prevention of non-steroidal anti-inflammatory drugs (NSAIDs) allergy, wherein the stable structure corresponding to the substance related to NSAIDs allergy is used as the substance for treating and/or preventing NSAIDs allergy.

Preferably, the stable structure corresponding to the substance refers to the structure of said substance that exists in a solid state at a high temperature of 500 °C or higher.

Preferably, the stable structure corresponding to the substance related to NSAIDs allergy is selected from one or more of the following:
(1) the stable structure corresponding to the substance that causes NSAIDs allergy in the organism;
(2) the stable structure corresponding to the substance of the organism itself and related to NSAIDs allergy;
(3) the stable structure corresponding to the substance that inhibits the structure that causes NSAIDs allergy;
(4) the stable structure corresponding to the substance containing a substance of any one of (1) to (3) above.

The substance for treating and/or preventing NSAIDs allergy designed according to the above-mentioned method.

The preparation method of the above-mentioned substance for treating and/or preventing NSAIDs allergy, wherein the stable structure corresponding to the substance related to NSAIDs allergy is prepared by a high-temperature carbonization method.

Use of the above-mentioned substance for treating and/or preventing NSAIDs allergy in the preparation of medicines, health products, foods, and food additives.

A method for designing substances for the treatment and/or prevention of chemical/chemical raw materials allergy, wherein the stable structure corresponding to the substance related to chemical/chemical raw materials allergy is used as the substance for treating and/or preventing chemical/chemical raw materials allergy.

Preferably, the stable structure corresponding to the substance refers to the structure of said substance that exists in a solid state at a high temperature of 500 °C or higher.

Preferably, the stable structure corresponding to the substance related to chemical/chemical raw materials allergy is selected from one or more of the following:
(1) the stable structure corresponding to the substance that causes chemical/chemical raw materials allergy in the organism;
(2) the stable structure corresponding to the substance of the organism itself and related to chemical/chemical raw materials allergy;
(3) the stable structure corresponding to the substance that inhibits the structure that causes chemical/chemical raw materials allergy;
(4) the stable structure corresponding to the substance containing a substance of any one of (1) to (3) above.

The substance for treating and/or preventing chemical/chemical raw materials allergy designed according to the above-mentioned method.

The preparation method of the above-mentioned substance for treating and/or preventing chemical/chemical raw materials allergy, wherein the stable structure corresponding to the substance related to chemical/chemical raw materials allergy is prepared by a high-temperature carbonization method.

Use of the above-mentioned substance for treating and/or preventing chemical/chemical raw materials allergy in the preparation of medicines, health products, foods, and food additives.

A method for designing substances for the treatment and/or prevention of Thiuram mix allergy, wherein the stable structure corresponding to the substance related to Thiuram mix allergy is used as the substance for treating and/or preventing Thiuram mix allergy.

Preferably, the stable structure corresponding to the substance refers to the structure of said substance that exists in a solid state at a high temperature of 500 °C or higher.

Preferably, the stable structure corresponding to the substance related to Thiuram mix allergy is selected from one or more of the following:
(1) the stable structure corresponding to the substance that causes Thiuram mix allergy in the organism;
(2) the stable structure corresponding to the substance of the organism itself and related to Thiuram mix allergy;
(3) the stable structure corresponding to the substance that inhibits the structure that causes Thiuram mix allergy;
(4) the stable structure corresponding to the substance containing a substance of any one of (1) to (3) above.

The substance for treating and/or preventing Thiuram mix allergy designed according to the above-mentioned method.

The preparation method of the above-mentioned substance for treating and/or preventing Thiuram mix allergy, wherein the stable structure corresponding to the substance related to Thiuram mix allergy is prepared by a high-temperature carbonization method.

Use of the above-mentioned substance for treating and/or preventing Thiuram mix allergy in the preparation of medicines, health products, foods, and food additives.

A method for designing substances for the treatment and/or prevention of formaldehyde allergy, wherein the stable structure corresponding to the substance related to formaldehyde allergy is used as the substance for treating and/or preventing formaldehyde allergy.

Preferably, the stable structure corresponding to the substance refers to the structure of said substance that exists in a solid state at a high temperature of 500 °C or higher.

Preferably, the stable structure corresponding to the substance related to formaldehyde allergy is selected from one or more of the following:
(1) the stable structure corresponding to the substance that causes formaldehyde allergy in the organism;
(2) the stable structure corresponding to the substance of the organism itself and related to formaldehyde allergy;
(3) the stable structure corresponding to the substance that inhibits the structure that causes formaldehyde allergy;
(4) the stable structure corresponding to the substance containing a substance of any one of (1) to (3) above.

The substance for treating and/or preventing formaldehyde allergy designed according to the above-mentioned method.

The preparation method of the above-mentioned substance for treating and/or preventing formaldehyde allergy, wherein the stable structure corresponding to the substance related to formaldehyde allergy is prepared by a high-temperature carbonization method.

Use of the above-mentioned substance for treating and/or preventing formaldehyde allergy in the preparation of medicines, health products, foods, and food additives.

A method for designing substances for the treatment and/or prevention of spice mixture allergy, wherein the stable structure corresponding to the substance related to spice mixture allergy is used as the substance for treating and/or preventing spice mixture allergy.

Preferably, the stable structure corresponding to the substance refers to the structure of said substance that exists in a solid state at a high temperature of 500 °C or higher.

Preferably, the stable structure corresponding to the substance related to spice mixture allergy is selected from one or more of the following:
(1) the stable structure corresponding to the substance that causes spice mixture allergy in the organism;
(2) the stable structure corresponding to the substance of the organism itself and related to spice mixture allergy;
(3) the stable structure corresponding to the substance that inhibits the structure that causes spice mixture allergy;
(4) the stable structure corresponding to the substance containing a substance of any one of (1) to (3) above.

The substance for treating and/or preventing spice mixture allergy designed according to the above-mentioned method.

The preparation method of the above-mentioned substance for treating and/or preventing spice mixture allergy, wherein the stable structure corresponding to the substance related to spice mixture allergy is prepared by a high-temperature carbonization method.

Use of the above-mentioned substance for treating and/or preventing spice mixture allergy in the preparation of medicines, health products, foods, and food additives.

A method for designing substances for the treatment and/or prevention of fragrance mix allergy, wherein the stable structure corresponding to the substance related to fragrance mix allergy is used as the substance for treating and/or preventing fragrance mix allergy.

Preferably, the stable structure corresponding to the substance refers to the structure of said substance that exists in a solid state at a high temperature of 500 °C or higher.

Preferably, the stable structure corresponding to the substance related to fragrance mix allergy is selected from one or more of the following:
(1) the stable structure corresponding to the substance that causes fragrance mix allergy in the organism;
(2) the stable structure corresponding to the substance of the organism itself and related to fragrance mix allergy;
(3) the stable structure corresponding to the substance that inhibits the structure that causes fragrance mix allergy;
(4) the stable structure corresponding to the substance containing a substance of any one of (1) to (3) above.

The substance for treating and/or preventing fragrance mix allergy designed according to the above-mentioned method.

The preparation method of the above-mentioned substance for treating and/or preventing fragrance mix allergy, wherein the stable structure corresponding to the substance related to fragrance mix allergy is prepared by a high-temperature carbonization method.

Use of the above-mentioned substance for treating and/or preventing fragrance mix allergy in the preparation of medicines, health products, foods, and food additives.

A method for designing substances for the treatment and/or prevention of p-phenylene diamine allergy, wherein the stable structure corresponding to the substance related to p-phenylene diamine allergy is used as the substance for treating and/or preventing p-phenylene diamine allergy.

Preferably, the stable structure corresponding to the substance refers to the structure of said substance that exists in a solid state at a high temperature of 500 °C or higher.

Preferably, the stable structure corresponding to the substance related to p-phenylene diamine allergy is selected from one or more of the following:
(1) the stable structure corresponding to the substance that causes p-phenylene diamine allergy in the organism;
(2) the stable structure corresponding to the substance of the organism itself and related to p-phenylene diamine allergy;
(3) the stable structure corresponding to the substance that inhibits the structure that causes p-phenylene diamine allergy;
(4) the stable structure corresponding to the substance containing a substance of any one of (1) to (3) above.

The substance for treating and/or preventing p-phenylene diamine allergy designed according to the above-mentioned method.

The preparation method of the above-mentioned substance for treating and/or preventing p-phenylene diamine allergy, wherein the stable structure corresponding to the substance related to p-phenylene diamine allergy is prepared by a high-temperature carbonization method.

Use of the above-mentioned substance for treating and/or preventing p-phenylene diamine allergy in the preparation of medicines, health products, foods, and food additives.

A method for designing substances for the treatment and/or prevention of Balsam of Peru allergy, wherein the stable structure corresponding to the substance related to Balsam of Peru allergy is used as the substance for treating and/or preventing Balsam of Peru allergy.

Preferably, the stable structure corresponding to the substance refers to the structure of said substance that exists in a solid state at a high temperature of 500 °C or higher.

Preferably, the stable structure corresponding to the substance related to Balsam of Peru allergy is selected from one or more of the following:
(1) the stable structure corresponding to the substance that causes Balsam of Peru allergy in the organism;
(2) the stable structure corresponding to the substance of the organism itself and related to Balsam of Peru allergy;
(3) the stable structure corresponding to the substance that inhibits the structure that causes Balsam of Peru allergy;
(4) the stable structure corresponding to the substance containing a substance of any one of (1) to (3) above.

The substance for treating and/or preventing Balsam of Peru allergy designed according to the above-mentioned method.

The preparation method of the above-mentioned substance for treating and/or preventing Balsam of Peru allergy, wherein the stable structure corresponding to the substance related to Balsam of Peru allergy is prepared by a high-temperature carbonization method.

Use of the above-mentioned substance for treating and/or preventing Balsam of Peru allergy in the preparation of medicines, health products, foods, and food additives.

A method for designing substances for the treatment and/or prevention of lanolin alcohol allergy, wherein the stable structure corresponding to the substance related to lanolin alcohol allergy is used as the substance for treating and/or preventing lanolin alcohol allergy.

Preferably, the stable structure corresponding to the substance refers to the structure of said substance that exists in a solid state at a high temperature of 500 °C or higher.

Preferably, the stable structure corresponding to the substance related to lanolin alcohol allergy is selected from one or more of the following:
(1) the stable structure corresponding to the substance that causes lanolin alcohol allergy in the organism;
(2) the stable structure corresponding to the substance of the organism itself and related to lanolin alcohol allergy;
(3) the stable structure corresponding to the substance that inhibits the structure that causes lanolin alcohol allergy;
(4) the stable structure corresponding to the substance containing a substance of any one of (1) to (3) above.

The substance for treating and/or preventing lanolin alcohol allergy designed according to the above-mentioned method.

The preparation method of the above-mentioned substance for treating and/or preventing lanolin alcohol allergy, wherein the stable structure corresponding to the substance related to lanolin alcohol allergy is prepared by a high-temperature carbonization method.

Use of the above-mentioned substance for treating and/or preventing lanolin alcohol allergy in the preparation of medicines, health products, foods, and food additives.

A method for designing substances for the treatment and/or prevention of chlorides allergy, wherein the stable structure corresponding to the substance related to chlorides allergy is used as the substance for treating and/or preventing chlorides allergy.

Preferably, the stable structure corresponding to the substance refers to the structure of said substance that exists in a solid state at a high temperature of 500 °C or higher.

Preferably, the stable structure corresponding to the substance related to chlorides allergy is selected from one or more of the following:
(1) the stable structure corresponding to the substance that causes chlorides allergy in the organism;
(2) the stable structure corresponding to the substance of the organism itself and related to chlorides allergy;
(3) the stable structure corresponding to the substance that inhibits the structure that causes chlorides allergy;
(4) the stable structure corresponding to the substance containing a substance of any one of (1) to (3) above.

The substance for treating and/or preventing chlorides allergy designed according to the above-mentioned method.

The preparation method of the above-mentioned substance for treating and/or preventing chlorides allergy, wherein the stable structure corresponding to the substance related to chlorides allergy is prepared by a high-temperature carbonization method.

Use of the above-mentioned substance for treating and/or preventing chlorides allergy in the preparation of medicines, health products, foods, and food additives.

A method for designing substances for the treatment and/or prevention of Kathon CG allergy, wherein the stable structure corresponding to the substance related to Kathon CG allergy is used as the substance for treating and/or preventing Kathon CG allergy.

Preferably, the stable structure corresponding to the substance refers to the structure of said substance that exists in a solid state at a high temperature of 500 °C or higher.

Preferably, the stable structure corresponding to the substance related to Kathon CG allergy is selected from one or more of the following:
(1) the stable structure corresponding to the substance that causes Kathon CG allergy in the organism;
(2) the stable structure corresponding to the substance of the organism itself and related to Kathon CG allergy;
(3) the stable structure corresponding to the substance that inhibits the structure that causes Kathon CG allergy;
(4) the stable structure corresponding to the substance containing a substance of any one of (1) to (3) above.

The substance for treating and/or preventing Kathon CG allergy designed according to the above-mentioned method.

The preparation method of the above-mentioned substance for treating and/or preventing Kathon CG allergy, wherein the stable structure corresponding to the substance related to Kathon CG allergy is prepared by a high-temperature carbonization method.

Use of the above-mentioned substance for treating and/or preventing Kathon CG allergy in the preparation of medicines, health products, foods, and food additives.

A method for designing substances for the treatment and/or prevention of carba mix allergy, wherein the stable structure corresponding to the substance related to carba mix allergy is used as the substance for treating and/or preventing carba mix allergy.

Preferably, the stable structure corresponding to the substance refers to the structure of said substance that exists in a solid state at a high temperature of 500 °C or higher.

Preferably, the stable structure corresponding to the substance related to carba mix allergy is selected from one or more of the following:
(1) the stable structure corresponding to the substance that causes carba mix allergy in the organism;
(2) the stable structure corresponding to the substance of the organism itself and related to carba mix allergy;
(3) the stable structure corresponding to the substance that inhibits the structure that causes carba mix allergy;
(4) the stable structure corresponding to the substance containing a substance of any one of (1) to (3) above.

The substance for treating and/or preventing carba mix allergy designed according to the above-mentioned method.

The preparation method of the above-mentioned substance for treating and/or preventing carba mix allergy, wherein the stable structure corresponding to the substance related to carba mix allergy is prepared by a high-temperature carbonization method.

Use of the above-mentioned substance for treating and/or preventing carba mix allergy in the preparation of medicines, health products, foods, and food additives.

A method for designing substances for the treatment and/or prevention of black rubber mix allergy, wherein the stable structure corresponding to the substance related to black rubber mix allergy is used as the substance for treating and/or preventing black rubber mix allergy.

Preferably, the stable structure corresponding to the substance refers to the structure of said substance that exists in a solid state at a high temperature of 500 °C or higher.

Preferably, the stable structure corresponding to the substance related to black rubber mix allergy is selected from one or more of the following:
(1) the stable structure corresponding to the substance that causes black rubber mix allergy in the organism;
(2) the stable structure corresponding to the substance of the organism itself and related to black rubber mix allergy;
(3) the stable structure corresponding to the substance that inhibits the structure that causes black rubber mix allergy;
(4) the stable structure corresponding to the substance containing a substance of any one of (1) to (3) above.

The substance for treating and/or preventing black rubber mix allergy designed according to the above-mentioned method.

The preparation method of the above-mentioned substance for treating and/or preventing black rubber mix allergy, wherein the stable structure corresponding to the substance related to black rubber mix allergy is prepared by a high-temperature carbonization method.

Use of the above-mentioned substance for treating and/or preventing black rubber mix allergy in the preparation of medicines, health products, foods, and food additives.

A method for designing substances for the treatment and/or prevention of p-tert-butyl phenol formaldehyde resin allergy, wherein the stable structure corresponding to the substance related to p-tert-butyl phenol formaldehyde resin allergy is used as the substance for treating and/or preventing p-tert-butyl phenol formaldehyde resin allergy.

Preferably, the stable structure corresponding to the substance refers to the structure of said substance that exists in a solid state at a high temperature of 500 °C or higher.

Preferably, the stable structure corresponding to the substance related to p-tert-butyl phenol formaldehyde resin allergy is selected from one or more of the following:
(1) the stable structure corresponding to the substance that causes p-tert-butyl phenol formaldehyde resin allergy in the organism;
(2) the stable structure corresponding to the substance of the organism itself and related to p-tert-butyl phenol formaldehyde resin allergy;
(3) the stable structure corresponding to the substance that inhibits the structure that causes p-tert-butyl phenol formaldehyde resin allergy;
(4) the stable structure corresponding to the substance containing a substance of any one of (1) to (3) above.

The substance for treating and/or preventing p-tert-butyl phenol formaldehyde resin allergy designed according to the above-mentioned method.

The preparation method of the above-mentioned substance for treating and/or preventing p-tert-butyl phenol formaldehyde resin allergy, wherein the stable structure corresponding to the substance related to p-tert-butyl phenol formaldehyde resin allergy is prepared by a high-temperature carbonization method.

Use of the above-mentioned substance for treating and/or preventing p-tert-butyl phenol formaldehyde resin allergy in the preparation of medicines, health products, foods, and food additives.

A method for designing substances for the treatment and/or prevention of Quaternium-15 allergy, wherein the stable structure corresponding to the substance related to Quaternium-15 allergy is used as the substance for treating and/or preventing Quaternium-15 allergy.

Preferably, the stable structure corresponding to the substance refers to the structure of said substance that exists in a solid state at a high temperature of 500 °C or higher.

Preferably, the stable structure corresponding to the substance related to Quaternium-15 allergy is selected from one or more of the following:
(1) the stable structure corresponding to the substance that causes Quaternium-15 allergy in the organism;
(2) the stable structure corresponding to the substance of the organism itself and related to Quaternium-15 allergy;
(3) the stable structure corresponding to the substance that inhibits the structure that causes Quaternium-15 allergy;
(4) the stable structure corresponding to the substance containing a substance of any one of (1) to (3) above.

The substance for treating and/or preventing Quaternium-15 allergy designed according to the above-mentioned method.

The preparation method of the above-mentioned substance for treating and/or preventing Quaternium-15 allergy, wherein the stable structure corresponding to the substance related to Quaternium-15 allergy is prepared by a high-temperature carbonization method.

Use of the above-mentioned substance for treating and/or preventing Quaternium-15 allergy in the preparation of medicines, health products, foods, and food additives.

A method for designing substances for the treatment and/or prevention of mercaptobenzothiazole allergy, wherein the stable structure corresponding to the substance related to mercaptobenzothiazole allergy is used as the substance for treating and/or preventing mercaptobenzothiazole allergy.

Preferably, the stable structure corresponding to the substance refers to the structure of said substance that exists in a solid state at a high temperature of 500 °C or higher.

Preferably, the stable structure corresponding to the substance related to mercaptobenzothiazole allergy is selected from one or more of the following:
(1) the stable structure corresponding to the substance that causes mercaptobenzothiazole allergy in the organism;
(2) the stable structure corresponding to the substance of the organism itself and related to mercaptobenzothiazole allergy;
(3) the stable structure corresponding to the substance that inhibits the structure that causes mercaptobenzothiazole allergy;
(4) the stable structure corresponding to the substance containing a substance of any one of (1) to (3) above.

The substance for treating and/or preventing mercaptobenzothiazole allergy designed according to the above-mentioned method.

The preparation method of the above-mentioned substance for treating and/or preventing mercaptobenzothiazole allergy, wherein the stable structure corresponding to the substance related to mercaptobenzothiazole allergy is prepared by a high-temperature carbonization method.

Use of the above-mentioned substance for treating and/or preventing mercaptobenzothiazole allergy in the preparation of medicines, health products, foods, and food additives.

A method for designing substances for the treatment and/or prevention of alcohol allergy, wherein the stable structure corresponding to the substance related to alcohol allergy is used as the substance for treating and/or preventing alcohol allergy.

Preferably, the stable structure corresponding to the substance refers to the structure of said substance that exists in a solid state at a high temperature of 500 °C or higher.

Preferably, the stable structure corresponding to the substance related to alcohol allergy is selected from one or more of the following:
(1) the stable structure corresponding to the substance that causes alcohol allergy in the organism;
(2) the stable structure corresponding to the substance of the organism itself and related to alcohol allergy;
(3) the stable structure corresponding to the substance that inhibits the structure that causes alcohol allergy;
(4) the stable structure corresponding to the substance containing a substance of any one of (1) to (3) above.

The substance for treating and/or preventing alcohol allergy designed according to the above-mentioned method.

The preparation method of the above-mentioned substance for treating and/or preventing alcohol allergy, wherein the stable structure corresponding to the substance related to alcohol allergy is prepared by a high-temperature carbonization method.

Use of the above-mentioned substance for treating and/or preventing alcohol allergy in the preparation of medicines, health products, foods, and food additives.

A method for designing substances for the treatment and/or prevention of mercapto mix allergy, wherein the stable structure corresponding to the substance related to mercapto mix allergy is used as the substance for treating and/or preventing mercapto mix allergy.

Preferably, the stable structure corresponding to the substance refers to the structure of said substance that exists in a solid state at a high temperature of 500 °C or higher.

Preferably, the stable structure corresponding to the substance related to mercapto mix allergy is selected from one or more of the following:
(1) the stable structure corresponding to the substance that causes mercapto mix allergy in the organism;
(2) the stable structure corresponding to the substance of the organism itself and related to mercapto mix allergy;
(3) the stable structure corresponding to the substance that inhibits the structure that causes mercapto mix allergy;
(4) the stable structure corresponding to the substance containing a substance of any one of (1) to (3) above.

The substance for treating and/or preventing mercapto mix allergy designed according to the above-mentioned method.

The preparation method of the above-mentioned substance for treating and/or preventing mercapto mix allergy, wherein the stable structure corresponding to the substance related to mercapto mix allergy is prepared by a high-temperature carbonization method.

Use of the above-mentioned substance for treating and/or preventing mercapto mix allergy in the preparation of medicines, health products, foods, and food additives.

The essence of health is the dynamic balance of the structural system, which is the result of the interactions between the structures in the structural system. The most fundamental interactions are promotion and restriction. In a biological structural system, if a structure lacks a structure that promotes it, this structure will be lacking, and if it lacks a structure that restricts it, this structure will be excessive, both of these situations will cause an imbalance in the structural system. The essence of disease is the imbalance of biological structure system. As can be seen from the above technical solutions, the method for treating or preventing diseases disclosed in the present invention uses the stable structure of the substance related to diseases as an external structure to act on the biological structural system (e.g. via oral administration), and the biological structural system regulates the gene or gene expression by recognizing and transmitting the structural information of the external structure and using its own self-adaptation, self-organization and other functions, so as to treat or prevent the diseases related to the substance.

It can be seen from the above technical solutions that the beneficial effects of the substances for treating and/or preventing diseases and their design methods and preparation methods disclosed in the present invention include:
1. The design method and the preparation method of the substance for treating and/or preventing diseases according to the present invention are not based on the concrete structure of the substance causing diseases or the specific pathogenic mechanism, but are characterized in that a stable structure corresponding to the substance which is the same as or similar to an external substance or a substance in the body that causes diseases is used as an external structure to act on a biological structural system, and said external structure enable organisms to eliminate or alleviate diseases or prevent the occurrence of diseases according to structural self-adaptation and self-organization. Therefore, the method of the present invention is versatile.
2. The present invention combines the concrete structure of Western medicine research and the macroscopic theory established by TCM, treats and/or prevents diseases from the perspective of a biological system. Relatively speaking, the system is limited, and the disease is unlimited, because different lesions of the same organ will cause different diseases, and the symptoms of the same disease in different individuals are not exactly the same. Whether in accordance with Western medicine or TCM, from a systematic point of view, people are the same, and even humans, pigs, cows, sheep, and primates are basically the same. Therefore, when designing substance for treating and/or preventing diseases based on the system, for diseases caused by internal diseases of organisms, only the specific diseased cells, tissues or organs need to be known, and there is no need to explore the pathogenic mechanism and specific symptoms of the disease. You only need to know the system where the disease-related substances are located or the cells, tissues or organs belonging to the system, and use the corresponding stable structure as an external structure to act on the biological structural system. The structural system of the organism enable the organism to establish the balance with the structure of the external substance or restore the structural imbalance in the biological structural system according to structural self-adaptation and self-organization, thereby eliminating, relieving the diseases or preventing the diseases.
3. The substance for treating and/or preventing diseases and design method and preparation method thereof according to the present invention avoid the research on the disease mechanism and greatly save the investment of time, manpower, material resources and financial resources. It greatly shortens the cycle of drug research and development and reduces the cost of disease treatment, and greatly simplifies drug research and development, disease diagnosis, and treatment.

### Detailed Description

The present invention discloses substances for treating and/or preventing allergic diseases, the design method and the preparation method thereof. Those skilled in the art can learn from the content of this article, use different materials, improve the process or adopt other similar processes for preparation, all of which are considered to be included in the present invention. In particular, it should be pointed out that all such substitutions and modifications will be apparent to those skilled in the art and are intended to be included herein. While the methods and products of the present invention have been described in terms of preferred embodiments, it will be apparent to those of ordinary skill in the art that variations and modifications of the methods described herein, as well as appropriate variations and combinations, may be made to implement and use the techniques of the present invention without departing from the spirit and scope of the invention.

In order to further understand the present invention, the technical solutions in the embodiments of the present invention will be clearly and completely described below with reference to the examples of the present invention, and it is obvious that the described examples are only a part of the embodiments of the present invention, and not all of the embodiments. All other embodiments, which can be obtained by a person skilled in the art without making any creative effort based on the embodiments in the present invention, belong to the protection scope of the present invention.

The specific embodiments are as follows:

### Example 1: Substance for treating and/or preventing shrimp allergy, and design method and preparation method thereof

Since shrimps contain substances causing shrimp allergy and are easily available, it is preferable to use the stable structure corresponding to shrimp as the substance for treating and/or preventing shrimp allergy. If other organs or tissues also contain the substance related to the shrimp allergy disease, it is also preferable to use the stable structure corresponding to the organ or tissue containing the substance related to the shrimp allergy disease as the substance for treating and/or preventing shrimp allergy, so that the complicated problems can be simplified, and the substance for treating and/or preventing shrimp allergy can be designed and prepared without knowing the specific allergen structure and pathogenic mechanism.

The shrimp is carbonized at high temperature to obtain the substance for treating and/or preventing shrimp allergy.

Using the same method, the present invention prepared a series of substances for treating and/or preventing allergic diseases caused by different food allergens.

### Example 2: Substance for treating and/or preventing Artemisia pollen allergy, and design method and preparation method thereof

Since Artemisia pollen contains substances causing Artemisia pollen allergy and is easily available, it is preferable to use the stable structure corresponding to Artemisia pollen as the substance for treating and/or preventing Artemisia pollen allergy. If other organs or tissues also contain the substance related to the Artemisia pollen allergy disease, it is also preferable to use the stable structure corresponding to the organ or tissue containing the substance related to the Artemisia pollen allergy disease as the substance for treating and/or preventing Artemisia pollen allergy, so that the complicated problems can be simplified, and the substance for treating and/or preventing Artemisia pollen allergy can be designed and prepared without knowing the specific allergen structure and pathogenic mechanism.

The Artemisia pollen is carbonized at high temperature to obtain the substance for treating and/or preventing Artemisia pollen allergy.

Using the same method, the present invention prepared a series of substances for treating and/or preventing biological allergic diseases caused by different pollen allergens.

### Example 3: Clinical application test of the substances prepared in Example 1 and Example 2 and various substances for treating allergic diseases prepared in the same manner in the treatment of allergic diseases

The present invention uses the substances prepared in the above Example 1 and Example 2 for efficacy verification, and the details are as follows:
1. **Clinical data:** Clinical validation was performed from May 2018 to May 2019. The specific process comprises the steps of hospitalizing the patient, self-describing symptoms by the pateint, carrying out allergy tests (skin test, blood test and the like) to confirm the allergen, carrying out targeted treatment for different allergens once. If there are too many allergens, a second targeted treatment will be carried out. After treatment, testing whether the patient was desensitized to the allergen by direct contact or consumption of the allergen, carrying out the same allergy tests, and further verifying the desensitization effect by comparing testing indexes. A total of 108 patients with allergies in the outpatient and inpatient departments were collected and randomly divided into treatment group and control group, with 54 patients in each group. There was no significant difference in clinical data between the two groups. Statistical analysis of age, disease course, gender, allergens, complications, etc. between the two groups showed that the differences were not significant (P>0.05), and they were comparable.
2. **Diagnostic criteria:** Refer to the "Guidelines for the European Society of Allergy and Clinical Immunology (2007)" and the "WAO Guidelines for the Diagnosis of Severe Allergic Reactions" for the diagnosis of allergies.
3. **Treatment method:** Patients in the treatment group were orally administered the substance for treating allergic diseases of the present invention once a day, 3 g/time, for 3 days continuously; patients in the control group were treated with conventional western medicine or desensitization therapy according to the doctor's instructions, including injection desensitization, sublingual desensitization, desensitization patch, antihistamine drugs, allergic reaction mediator blockers, corticosteroids, calcium agents, immunosuppressants, etc.
4. **Criteria for judging the treatment effect:** The treatment effect of the disease is evaluated according to the symptoms, and the total effective rate = cure rate + marked improvement rate + effective rate, wherein "cure" means that the clinical symptoms of allergy of the patient completely disappeared after treatment, no allergic reaction occurs after the patient contacts with the allergen again, and no recurrence occurs within 1 year of follow-up; "marked improvement" means that the clinical symptoms of the patient are significantly improved after treatment, no allergic reaction occurs after the patient contacts with the allergen again, and recurrence occurs within 1 year of follow-up; "effective" means that the clinical symptoms of the patient are relieved to some extent after treatment, and compared with before treatment, allergic symptoms are alleviated when exposed to allergens again; "ineffective" means that the above clinical symptoms and manifestations of the patient have not changed significantly or worsened after treatment.
5. **Efficacy results**: As shown in Table 1, the overall treatment effect of the control group was "marked improvement" in 2 cases, "effective" in 17 cases, and "ineffective" in 35 cases. More than 95% of patients still have allergic symptoms after being exposed to allergens again. In all 3 patients treated with desensitization therapy, the treatment was terminated early because of obvious intolerance. In the control group, there were 27 cases of adverse drug reactions and 14 cases of drug tolerance. The drugs needed to be changed or increased doses according to doctor's orders.

In the treatment group, 52 patients were cured, with a total effective rate of 96.30%, and the patients did not develop allergic symptoms after repeated exposure to allergens. At the same time, there were no side effects and drug dependence in the treatment group during the observation period.

**TABLE 1. Comparison of the treatment effect of allergic patients (cases)**

| Group | Number of cases | Cure | Marked improvement | Effective | Ineffective | Total effective rate (%) |
|---|---|---|---|---|---|---|
| Treatment group | 54 | 52 | 0 | 0 | 2 | 96.30 |
| Control group | 54 | 0 | 2 | 17 | 35 | 35.19 |

## Claims

1. A substance for treating and/or preventing allergic diseases and its design method and preparation method, **characterized in that**:
the stable structure corresponding to the substance related to the allergic disease is used as the substance for treating and/or preventing said allergic disease; and
the stable structure corresponding to the substance is **characterized by** the structure of said substance that exists in a solid state at a high temperature of 500 °C or higher.

2. The method according to claim 1, **characterized in that** said stable structure corresponding to the substance related to the allergic disease is selected from one or more of the following:
(1) the stable structure corresponding to the substance that causes allergic disease in the organism;
(2) the stable structure corresponding to the substance of the organism itself and related to allergic disease;
(3) the stable structure corresponding to the substance that inhibits the structure that causes allergic disease;
(4) the stable structure corresponding to the substance containing a substance of any one of (1) to (3) above.

3. A substance for treating and/or preventing said diseases designed according to the method of any one of claims 1-2.

4. The preparation method of the substance for treating and/or preventing allergic disease according to claim 3, **characterized in that** the stable structure corresponding to the substance is prepared by a high-temperature carbonization method.

5. A method for designing substances for treating and/or preventing food allergic diseases, **characterized in that**:
the stable structure corresponding to the substance related to the food allergic disease is used as the substance for treating and/or preventing the food allergic disease; and
the stable structure corresponding to the substance is **characterized by** the structure of said substance that exists in a solid state at a high temperature of 500 °C or higher.

6. The method according to claim 1, **characterized in that** said stable structure corresponding to the substance related to the allergic disease is selected from one or more of the following:
(1) the stable structure corresponding to the substance that causes allergic disease in the organism;
(2) the stable structure corresponding to the substance of the organism itself and related to allergic disease;
(3) the stable structure corresponding to the substance that inhibits the structure that causes allergic disease;
(4) the stable structure corresponding to the substance containing a substance of any one of (1) to (3) above.

7. A substance for treating and/or preventing food allergic diseases designed according to the method of any one of claims 5-6.

8. The preparation method of the substance for treating and/or preventing food allergic disease according to claim 7, **characterized in that** the stable structure corresponding to the substance is prepared by a high-temperature carbonization method.

9. A method for designing substances for treating and/or preventing peanut allergy, **characterized in that**:
the stable structure corresponding to the substance related to peanut allergy is used as the substance for treating and/or preventing peanut allergy; and
the stable structure corresponding to the substance is **characterized by** the structure of said substance that exists in a solid state at a high temperature of 500 °C or higher.

10. The method according to claim 9, **characterized in that** said stable structure corresponding to the substance related to the allergic disease is selected from one or more of the following:
(1) the stable structure corresponding to the substance that causes peanut allergy in the organism;
(2) the stable structure corresponding to the substance of the organism itself and related to peanut allergy;
(3) the stable structure corresponding to the substance that inhibits the structure that causes peanut allergy;
(4) the stable structure corresponding to the substance containing a substance of any one of (1) to (3) above.

11. A substance for treating and/or preventing peanut allergy designed according to the method of any one of claims 8-9.

12. The preparation method of the substance for treating and/or preventing peanut allergy according to claim 11, **characterized in that** the stable structure corresponding to the substance is prepared by a high-temperature carbonization method.

13. A method for designing substances for treating and/or preventing egg allergy, **characterized in that**:
the stable structure corresponding to the substance related to egg allergy is used as the substance for treating and/or preventing egg allergy; and
the stable structure corresponding to the substance is **characterized by** the structure of said substance that exists in a solid state at a high temperature of 500 °C or higher.

14. The method according to claim 13, **characterized in that** said stable structure corresponding to the substance related to the allergic disease is selected from one or more of the following:
(1) the stable structure corresponding to the substance that causes egg allergy in the organism;
(2) the stable structure corresponding to the substance of the organism itself and related to egg allergy;
(3) the stable structure corresponding to the substance that inhibits the structure that causes egg allergy;
(4) the stable structure corresponding to the substance containing a substance of any one of (1) to (3) above.

15. A substance for treating and/or preventing egg allergy designed according to the method of any one of claims 13-14.

16. The preparation method of the substance for treating and/or preventing egg allergy according to claim 15, **characterized in that** the stable structure corresponding to the substance is prepared by a high-temperature carbonization method.

17. A method for designing substances for treating and/or preventing milk allergy, **characterized in that**:
the stable structure corresponding to the substance related to milk allergy is used as the substance for treating and/or preventing milk allergy; and
the stable structure corresponding to the substance is **characterized by** the structure of said substance that exists in a solid state at a high temperature of 500 °C or higher.

18. The method according to claim 17, **characterized in that** said stable structure corresponding to the substance related to the allergic disease is selected from one or more of the following:
(1) the stable structure corresponding to the substance that causes milk allergy in the organism;
(2) the stable structure corresponding to the substance of the organism itself and related to milk allergy;
(3) the stable structure corresponding to the substance that inhibits the structure that causes milk allergy;
(4) the stable structure corresponding to the substance containing a substance of any one of (1) to (3) above.

19. A substance for treating and/or preventing milk allergy designed according to the method of any one of claims 17-18.

20. The preparation method of the substance for treating and/or preventing milk allergy according to claim 19, **characterized in that** the stable structure corresponding to the substance is prepared by a high-temperature carbonization method.

21. A method for designing substances for treating and/or preventing soybean allergy, **characterized in that**:
the stable structure corresponding to the substance related to soybean allergy is used as the substance for treating and/or preventing soybean allergy; and
the stable structure corresponding to the substance is **characterized by** the structure of said substance that exists in a solid state at a high temperature of 500 °C or higher.

22. The method according to claim 21, **characterized in that** said stable structure corresponding to the substance related to the allergic disease is selected from one or more of the following:
(1) the stable structure corresponding to the substance that causes soybean allergy in the organism;
(2) the stable structure corresponding to the substance of the organism itself and related to soybean allergy;
(3) the stable structure corresponding to the substance that inhibits the structure that causes soybean allergy;
(4) the stable structure corresponding to the substance containing a substance of any one of (1) to (3) above.

23. A substance for treating and/or preventing soybean allergy designed according to the method of any one of claims 21-22.

24. The preparation method of the substance for treating and/or preventing soybean allergy according to claim 23, **characterized in that** the stable structure corresponding to the substance is prepared by a high-temperature carbonization method.

25. A method for designing substances for treating and/or preventing nuts allergy, **characterized in that**:
the stable structure corresponding to the substance related to nuts allergy is used as the substance for treating and/or preventing nuts allergy; and
the stable structure corresponding to the substance is **characterized by** the structure of said substance that exists in a solid state at a high temperature of 500 °C or higher.

26. The method according to claim 25, **characterized in that** said stable structure corresponding to the substance related to the allergic disease is selected from one or more of the following:
(1) the stable structure corresponding to the substance that causes nuts allergy in the organism;
(2) the stable structure corresponding to the substance of the organism itself and related to nuts allergy;
(3) the stable structure corresponding to the substance that inhibits the structure that causes nuts allergy;
(4) the stable structure corresponding to the substance containing a substance of any one of (1) to (3) above.

27. A substance for treating and/or preventing nuts allergy designed according to the method of any one of claims 25-26.

28. The preparation method of the substance for treating and/or preventing nuts allergy according to claim 27, **characterized in that** the stable structure corresponding to the substance is prepared by a high-temperature carbonization method.

29. A method for designing substances for treating and/or preventing fish allergy, **characterized in that**:
the stable structure corresponding to the substance related to fish allergy is used as the substance for treating and/or preventing fish allergy; and
the stable structure corresponding to the substance is **characterized by** the structure of said substance that exists in a solid state at a high temperature of 500 °C or higher.

30. The method according to claim 29, **characterized in that** said stable structure corresponding to the substance related to the allergic disease is selected from one or more of the following:
(1) the stable structure corresponding to the substance that causes fish allergy in the organism;
(2) the stable structure corresponding to the substance of the organism itself and related to fish allergy;
(3) the stable structure corresponding to the substance that inhibits the structure that causes fish allergy;
(4) the stable structure corresponding to the substance containing a substance of any one of (1) to (3) above.

31. A substance for treating and/or preventing fish allergy designed according to the method of any one of claims 29-30.

32. The preparation method of the substance for treating and/or preventing fish allergy according to claim 31, **characterized in that** the stable structure corresponding to the substance is prepared by a high-temperature carbonization method.

33. A method for designing substances for treating and/or preventing crustacean allergy, **characterized in that**:
the stable structure corresponding to the substance related to crustacean allergy is used as the substance for treating and/or preventing crustacean allergy; and
the stable structure corresponding to the substance is **characterized by** the structure of said substance that exists in a solid state at a high temperature of 500 °C or higher.

34. The method according to claim 33, **characterized in that** said stable structure corresponding to the substance related to the allergic disease is selected from one or more of the following:
(1) the stable structure corresponding to the substance that causes crustacean allergy in the organism;
(2) the stable structure corresponding to the substance of the organism itself and related to crustacean allergy;
(3) the stable structure corresponding to the substance that inhibits the structure that causes crustacean allergy;
(4) the stable structure corresponding to the substance containing a substance of any one of (1) to (3) above.

35. A substance for treating and/or preventing crustacean allergy designed according to the method of any one of claims 33-34.

36. The preparation method of the substance for treating and/or preventing crustacean allergy according to claim 35, **characterized in that** the stable structure corresponding to the substance is prepared by a high-temperature carbonization method.

37. A method for designing substances for treating and/or preventing wheat allergy, **characterized in that**:
the stable structure corresponding to the substance related to wheat allergy is used as the substance for treating and/or preventing wheat allergy; and
the stable structure corresponding to the substance is **characterized by** the structure of said substance that exists in a solid state at a high temperature of 500 °C or higher.

38. The method according to claim 37, **characterized in that** said stable structure corresponding to the substance related to the allergic disease is selected from one or more of the following:
(1) the stable structure corresponding to the substance that causes wheat allergy in the organism;
(2) the stable structure corresponding to the substance of the organism itself and related to wheat allergy;
(3) the stable structure corresponding to the substance that inhibits the structure that causes wheat allergy;
(4) the stable structure corresponding to the substance containing a substance of any one of (1) to (3) above.

39. A substance for treating and/or preventing wheat allergy designed according to the method of any one of claims 37-38.

40. The preparation method of the substance for treating and/or preventing wheat allergy according to claim 39, **characterized in that** the stable structure corresponding to the substance is prepared by a high-temperature carbonization method.

41. A method for designing substances for treating and/or preventing shrimp allergy, **characterized in that**:
the stable structure corresponding to the substance related to shrimp allergy is used as the substance for treating and/or preventing shrimp allergy; and
the stable structure corresponding to the substance is **characterized by** the structure of said substance that exists in a solid state at a high temperature of 500 °C or higher.

42. The method according to claim 41, **characterized in that** said stable structure corresponding to the substance related to the allergic disease is selected from one or more of the following:
(1) the stable structure corresponding to the substance that causes shrimp allergy in the organism;
(2) the stable structure corresponding to the substance of the organism itself and related to shrimp allergy;
(3) the stable structure corresponding to the substance that inhibits the structure that causes shrimp allergy;
(4) the stable structure corresponding to the substance containing a substance of any one of (1) to (3) above.

43. A substance for treating and/or preventing shrimp allergy designed according to the method of any one of claims 41-42.

44. The preparation method of the substance for treating and/or preventing shrimp allergy according to claim 43, **characterized in that** the stable structure corresponding to the substance is prepared by a high-temperature carbonization method.

45. A method for designing substances for treating and/or preventing fruit allergy, **characterized in that**:
the stable structure corresponding to the substance related to fruit allergy is used as the substance for treating and/or preventing fruit allergy; and
the stable structure corresponding to the substance is **characterized by** the structure of said substance that exists in a solid state at a high temperature of 500 °C or higher.

46. The method according to claim 45, **characterized in that** said stable structure corresponding to the substance related to the allergic disease is selected from one or more of the following:
(1) the stable structure corresponding to the substance that causes fruit allergy in the organism;
(2) the stable structure corresponding to the substance of the organism itself and related to fruit allergy;
(3) the stable structure corresponding to the substance that inhibits the structure that causes fruit allergy;
(4) the stable structure corresponding to the substance containing a substance of any one of (1) to (3) above.

47. A substance for treating and/or preventing fruit allergy designed according to the method of any one of claims 45-46.

48. The preparation method of the substance for treating and/or preventing fruit allergy according to claim 47, **characterized in that** the stable structure corresponding to the substance is prepared by a high-temperature carbonization method.

49. A method for designing substances for treating and/or preventing vegetable allergy, **characterized in that**:
the stable structure corresponding to the substance related to vegetable allergy is used as the substance for treating and/or preventing vegetable allergy; and
the stable structure corresponding to the substance is **characterized by** the structure of said substance that exists in a solid state at a high temperature of 500 °C or higher.

50. The method according to claim 49, **characterized in that** said stable structure corresponding to the substance related to the allergic disease is selected from one or more of the following:
(1) the stable structure corresponding to the substance that causes vegetable allergy in the organism;
(2) the stable structure corresponding to the substance of the organism itself and related to vegetable allergy;
(3) the stable structure corresponding to the substance that inhibits the structure that causes vegetable allergy;
(4) the stable structure corresponding to the substance containing a substance of any one of (1) to (3) above.

51. A substance for treating and/or preventing vegetable allergy designed according to the method of any one of claims 49-50.

52. The preparation method of the substance for treating and/or preventing vegetable allergy according to claim 51, **characterized in that** the stable structure corresponding to the substance is prepared by a high-temperature carbonization method.

53. A method for designing substances for treating and/or preventing plant seed allergy, **characterized in that**:
the stable structure corresponding to the substance related to plant seed allergy is used as the substance for treating and/or preventing plant seed allergy; and
the stable structure corresponding to the substance is **characterized by** the structure of said substance that exists in a solid state at a high temperature of 500 °C or higher.

54. The method according to claim 53, **characterized in that** said stable structure corresponding to the substance related to the allergic disease is selected from one or more of the following:
(1) the stable structure corresponding to the substance that causes plant seed allergy in the organism;
(2) the stable structure corresponding to the substance of the organism itself and related to plant seed allergy;
(3) the stable structure corresponding to the substance that inhibits the structure that causes plant seed allergy;
(4) the stable structure corresponding to the substance containing a substance of any one of (1) to (3) above.

55. A substance for treating and/or preventing plant seed allergy designed according to the method of any one of claims 53-54.

56. The preparation method of the substance for treating and/or preventing plant seed allergy according to claim 55, **characterized in that** the stable structure corresponding to the substance is prepared by a high-temperature carbonization method.

57. A method for designing substances for treating and/or preventing food additive allergy, **characterized in that**:
the stable structure corresponding to the substance related to food additive allergy is used as the substance for treating and/or preventing food additive allergy; and
the stable structure corresponding to the substance is **characterized by** the structure of said substance that exists in a solid state at a high temperature of 500 °C or higher.

58. The method according to claim 57, **characterized in that** said stable structure corresponding to the substance related to the allergic disease is selected from one or more of the following:
(1) the stable structure corresponding to the substance that causes food additive allergy in the organism;
(2) the stable structure corresponding to the substance of the organism itself and related to food additive allergy;
(3) the stable structure corresponding to the substance that inhibits the structure that causes food additive allergy;
(4) the stable structure corresponding to the substance containing a substance of any one of (1) to (3) above.

59. A substance for treating and/or preventing food additive allergy designed according to the method of any one of claims 57-58.

60. The preparation method of the substance for treating and/or preventing food additive allergy according to claim 59, **characterized in that** the stable structure corresponding to the substance is prepared by a high-temperature carbonization method.

61. A method for designing substances for treating and/or preventing meat allergy, **characterized in that**:
the stable structure corresponding to the substance related to meat allergy is used as the substance for treating and/or preventing meat allergy; and
the stable structure corresponding to the substance is **characterized by** the structure of said substance that exists in a solid state at a high temperature of 500 °C or higher.

62. The method according to claim 61, **characterized in that** said stable structure corresponding to the substance related to the allergic disease is selected from one or more of the following:
(1) the stable structure corresponding to the substance that causes meat allergy in the organism;
(2) the stable structure corresponding to the substance of the organism itself and related to meat allergy;
(3) the stable structure corresponding to the substance that inhibits the structure that causes meat allergy;
(4) the stable structure corresponding to the substance containing a substance of any one of (1) to (3) above.

63. A substance for treating and/or preventing meat allergy designed according to the method of any one of claims 61-62.

64. The preparation method of the substance for treating and/or preventing meat allergy according to claim 63, **characterized in that** the stable structure corresponding to the substance is prepared by a high-temperature carbonization method.

65. A method for designing substances for treating and/or preventing alcohol allergy, **characterized in that**:
the stable structure corresponding to the substance related to alcohol allergy is used as the substance for treating and/or preventing alcohol allergy; and
the stable structure corresponding to the substance is **characterized by** the structure of said substance that exists in a solid state at a high temperature of 500 °C or higher.

66. The method according to claim 65, **characterized in that** said stable structure corresponding to the substance related to the allergic disease is selected from one or more of the following:
(1) the stable structure corresponding to the substance that causes alcohol allergy in the organism;
(2) the stable structure corresponding to the substance of the organism itself and related to alcohol allergy;
(3) the stable structure corresponding to the substance that inhibits the structure that causes alcohol allergy;
(4) the stable structure corresponding to the substance containing a substance of any one of (1) to (3) above.

67. A substance for treating and/or preventing alcohol allergy designed according to the method of any one of claims 65-66.

68. The preparation method of the substance for treating and/or preventing alcohol allergy according to claim 67, **characterized in that** the stable structure corresponding to the substance is prepared by a high-temperature carbonization method.

69. A method for designing substances for treating and/or preventing room/house dust mite allergy, **characterized in that**:
the stable structure corresponding to the substance related to room/house dust mite allergy is used as the substance for treating and/or preventing room/house dust mite allergy; and
the stable structure corresponding to the substance is **characterized by** the structure of said substance that exists in a solid state at a high temperature of 500 °C or higher.

70. The method according to claim 69, **characterized in that** said stable structure corresponding to the substance related to the allergic disease is selected from one or more of the following:
(1) the stable structure corresponding to the substance that causes room/house dust mite allergy in the organism;
(2) the stable structure corresponding to the substance of the organism itself and related to room/house dust mite allergy;
(3) the stable structure corresponding to the substance that inhibits the structure that causes room/house dust mite allergy;
(4) the stable structure corresponding to the substance containing a substance of any one of (1) to (3) above.

71. A substance for treating and/or preventing room/house dust mite allergy designed according to the method of any one of claims 69-70.

72. The preparation method of the substance for treating and/or preventing room/house dust mite allergy according to claim 71, **characterized in that** the stable structure corresponding to the substance is prepared by a high-temperature carbonization method.

73. A method for designing substances for treating and/or preventing dust allergy, **characterized in that**:
the stable structure corresponding to the substance related to dust allergy is used as the substance for treating and/or preventing dust allergy; and
the stable structure corresponding to the substance is **characterized by** the structure of said substance that exists in a solid state at a high temperature of 500 °C or higher.

74. The method according to claim 73, **characterized in that** said stable structure corresponding to the substance related to the allergic disease is selected from one or more of the following:
(1) the stable structure corresponding to the substance that causes dust allergy in the organism;
(2) the stable structure corresponding to the substance of the organism itself and related to dust allergy;
(3) the stable structure corresponding to the substance that inhibits the structure that causes dust allergy;
(4) the stable structure corresponding to the substance containing a substance of any one of (1) to (3) above.

75. A substance for treating and/or preventing dust allergy designed according to the method of any one of claims 73-74.

76. The preparation method of the substance for treating and/or preventing dust allergy according to claim 75, **characterized in that** the stable structure corresponding to the substance is prepared by a high-temperature carbonization method.

77. A method for designing substances for treating and/or preventing fungal allergy, **characterized in that**:
the stable structure corresponding to the substance related to fungal allergy is used as the substance for treating and/or preventing fungal allergy; and
the stable structure corresponding to the substance is **characterized by** the structure of said substance that exists in a solid state at a high temperature of 500 °C or higher.

78. The method according to claim 77, **characterized in that** said stable structure corresponding to the substance related to the allergic disease is selected from one or more of the following:
(1) the stable structure corresponding to the substance that causes fungal allergy in the organism;
(2) the stable structure corresponding to the substance of the organism itself and related to fungal allergy;
(3) the stable structure corresponding to the substance that inhibits the structure that causes fungal allergy;
(4) the stable structure corresponding to the substance containing a substance of any one of (1) to (3) above.

79. A substance for treating and/or preventing fungal allergy designed according to the method of any one of claims 77-78.

80. The preparation method of the substance for treating and/or preventing fungal allergy according to claim 79, **characterized in that** the stable structure corresponding to the substance is prepared by a high-temperature carbonization method.

81. A method for designing substances for treating and/or preventing pollen allergy, **characterized in that**:
the stable structure corresponding to the substance related to pollen allergy is used as the substance for treating and/or preventing pollen allergy; and
the stable structure corresponding to the substance is **characterized by** the structure of said substance that exists in a solid state at a high temperature of 500 °C or higher.

82. The method according to claim 81, **characterized in that** said stable structure corresponding to the substance related to the allergic disease is selected from one or more of the following:
(1) the stable structure corresponding to the substance that causes pollen allergy in the organism;
(2) the stable structure corresponding to the substance of the organism itself and related to pollen allergy;
(3) the stable structure corresponding to the substance that inhibits the structure that causes pollen allergy;
(4) the stable structure corresponding to the substance containing a substance of any one of (1) to (3) above.

83. A substance for treating and/or preventing pollen allergy designed according to the method of any one of claims 81-82.

84. The preparation method of the substance for treating and/or preventing pollen allergy according to claim 83, **characterized in that** the stable structure corresponding to the substance is prepared by a high-temperature carbonization method.

85. A method for designing substances for treating and/or preventing ragweed pollen allergy, **characterized in that**:
the stable structure corresponding to the substance related to ragweed pollen allergy is used as the substance for treating and/or preventing ragweed pollen allergy; and
the stable structure corresponding to the substance is **characterized by** the structure of said substance that exists in a solid state at a high temperature of 500 °C or higher.

86. The method according to claim 85, **characterized in that** said stable structure corresponding to the substance related to the allergic disease is selected from one or more of the following:
(1) the stable structure corresponding to the substance that causes ragweed pollen allergy in the organism;
(2) the stable structure corresponding to the substance of the organism itself and related to ragweed pollen allergy;
(3) the stable structure corresponding to the substance that inhibits the structure that causes ragweed pollen allergy;
(4) the stable structure corresponding to the substance containing a substance of any one of (1) to (3) above.

87. A substance for treating and/or preventing ragweed pollen allergy designed according to the method of any one of claims 85-86.

88. The preparation method of the substance for treating and/or preventing ragweed pollen allergy according to claim 87, **characterized in that** the stable structure corresponding to the substance is prepared by a high-temperature carbonization method.

89. A method for designing substances for treating and/or preventing birch pollen allergy, **characterized in that**:
the stable structure corresponding to the substance related to birch pollen allergy is used as the substance for treating and/or preventing birch pollen allergy; and
the stable structure corresponding to the substance is **characterized by** the structure of said substance that exists in a solid state at a high temperature of 500 °C or higher.

90. The method according to claim 89, **characterized in that** said stable structure corresponding to the substance related to the allergic disease is selected from one or more of the following:
(1) the stable structure corresponding to the substance that causes birch pollen allergy in the organism;
(2) the stable structure corresponding to the substance of the organism itself and related to birch pollen allergy;
(3) the stable structure corresponding to the substance that inhibits the structure that causes birch pollen allergy;
(4) the stable structure corresponding to the substance containing a substance of any one of (1) to (3) above.

91. A substance for treating and/or preventing birch pollen allergy designed according to the method of any one of claims 89-90.

92. The preparation method of the substance for treating and/or preventing birch pollen allergy according to claim 91, **characterized in that** the stable structure corresponding to the substance is prepared by a high-temperature carbonization method.

93. A method for designing substances for treating and/or preventing Artemisia pollen allergy, **characterized in that**:
the stable structure corresponding to the substance related to Artemisia pollen allergy is used as the substance for treating and/or preventing Artemisia pollen allergy; and
the stable structure corresponding to the substance is **characterized by** the structure of said substance that exists in a solid state at a high temperature of 500 °C or higher.

94. The method according to claim 93, **characterized in that** said stable structure corresponding to the substance related to the allergic disease is selected from one or more of the following:
(1) the stable structure corresponding to the substance that causes Artemisia pollen allergy in the organism;
(2) the stable structure corresponding to the substance of the organism itself and related to Artemisia pollen allergy;
(3) the stable structure corresponding to the substance that inhibits the structure that causes Artemisia pollen allergy;
(4) the stable structure corresponding to the substance containing a substance of any one of (1) to (3) above.

95. A substance for treating and/or preventing Artemisia pollen allergy designed according to the method of any one of claims 93-94.

96. The preparation method of the substance for treating and/or preventing Artemisia pollen allergy according to claim 95, **characterized in that** the stable structure corresponding to the substance is prepared by a high-temperature carbonization method.

97. A method for designing substances for treating and/or preventing cedar pollen allergy, **characterized in that**:
the stable structure corresponding to the substance related to cedar pollen allergy is used as the substance for treating and/or preventing cedar pollen allergy; and
the stable structure corresponding to the substance is **characterized by** the structure of said substance that exists in a solid state at a high temperature of 500 °C or higher.

98. The method according to claim 97, **characterized in that** said stable structure corresponding to the substance related to the allergic disease is selected from one or more of the following:
(1) the stable structure corresponding to the substance that causes cedar pollen allergy in the organism;
(2) the stable structure corresponding to the substance of the organism itself and related to cedar pollen allergy;
(3) the stable structure corresponding to the substance that inhibits the structure that causes cedar pollen allergy;
(4) the stable structure corresponding to the substance containing a substance of any one of (1) to (3) above.

99. A substance for treating and/or preventing cedar pollen allergy designed according to the method of any one of claims 97-98.

100. The preparation method of the substance for treating and/or preventing cedar pollen allergy according to claim 99, **characterized in that** the stable structure corresponding to the substance is prepared by a high-temperature carbonization method.

101. A method for designing substances for treating and/or preventing maple pollen allergy, **characterized in that**:
the stable structure corresponding to the substance related to maple pollen allergy is used as the substance for treating and/or preventing maple pollen allergy; and
the stable structure corresponding to the substance is **characterized by** the structure of said substance that exists in a solid state at a high temperature of 500 °C or higher.

102. The method according to claim 101, **characterized in that** said stable structure corresponding to the substance related to the allergic disease is selected from one or more of the following:
(1) the stable structure corresponding to the substance that causes maple pollen allergy in the organism;
(2) the stable structure corresponding to the substance of the organism itself and related to maple pollen allergy;
(3) the stable structure corresponding to the substance that inhibits the structure that causes maple pollen allergy;
(4) the stable structure corresponding to the substance containing a substance of any one of (1) to (3) above.

103. A substance for treating and/or preventing maple pollen allergy designed according to the method of any one of claims 101-102.

104. The preparation method of the substance for treating and/or preventing maple pollen allergy according to claim 103, **characterized in that** the stable structure corresponding to the substance is prepared by a high-temperature carbonization method.

105. A method for designing substances for treating and/or preventing humulus pollen allergy, **characterized in that**:
the stable structure corresponding to the substance related to humulus pollen allergy is used as the substance for treating and/or preventing humulus pollen allergy; and
the stable structure corresponding to the substance is **characterized by** the structure of said substance that exists in a solid state at a high temperature of 500 °C or higher.

106. The method according to claim 105, **characterized in that** said stable structure corresponding to the substance related to the allergic disease is selected from one or more of the following:
(1) the stable structure corresponding to the substance that causes humulus pollen allergy in the organism;
(2) the stable structure corresponding to the substance of the organism itself and related to humulus pollen allergy;
(3) the stable structure corresponding to the substance that inhibits the structure that causes humulus pollen allergy;
(4) the stable structure corresponding to the substance containing a substance of any one of (1) to (3) above.

107. A substance for treating and/or preventing humulus pollen allergy designed according to the method of any one of claims 105-106.

108. The preparation method of the substance for treating and/or preventing humulus pollen allergy according to claim 107, **characterized in that** the stable structure corresponding to the substance is prepared by a high-temperature carbonization method.

109. A method for designing substances for treating and/or preventing willow catkins allergy, **characterized in that**:
the stable structure corresponding to the substance related to willow catkins allergy is used as the substance for treating and/or preventing willow catkins allergy; and
the stable structure corresponding to the substance is **characterized by** the structure of said substance that exists in a solid state at a high temperature of 500 °C or higher.

110. The method according to claim 109, **characterized in that** said stable structure corresponding to the substance related to the allergic disease is selected from one or more of the following:
(1) the stable structure corresponding to the substance that causes willow catkins allergy in the organism;
(2) the stable structure corresponding to the substance of the organism itself and related to willow catkins allergy;
(3) the stable structure corresponding to the substance that inhibits the structure that causes willow catkins allergy;
(4) the stable structure corresponding to the substance containing a substance of any one of (1) to (3) above.

111. A substance for treating and/or preventing willow catkins allergy designed according to the method of any one of claims 109-110.

112. The preparation method of the substance for treating and/or preventing willow catkins allergy according to claim 111, **characterized in that** the stable structure corresponding to the substance is prepared by a high-temperature carbonization method.

113. A method for designing substances for treating and/or preventing sycamore pollen allergy, **characterized in that**:
the stable structure corresponding to the substance related to sycamore pollen allergy is used as the substance for treating and/or preventing sycamore pollen allergy; and
the stable structure corresponding to the substance is **characterized by** the structure of said substance that exists in a solid state at a high temperature of 500 °C or higher.

114. The method according to claim 113, **characterized in that** said stable structure corresponding to the substance related to the allergic disease is selected from one or more of the following:
(1) the stable structure corresponding to the substance that causes sycamore pollen allergy in the organism;
(2) the stable structure corresponding to the substance of the organism itself and related to sycamore pollen allergy;
(3) the stable structure corresponding to the substance that inhibits the structure that causes sycamore pollen allergy;
(4) the stable structure corresponding to the substance containing a substance of any one of (1) to (3) above.

115. A substance for treating and/or preventing sycamore pollen allergy designed according to the method of any one of claims 113-114.

116. The preparation method of the substance for treating and/or preventing sycamore pollen allergy according to claim 115, **characterized in that** the stable structure corresponding to the substance is prepared by a high-temperature carbonization method.

117. A method for designing substances for treating and/or preventing ginkgo pollen allergy, **characterized in that**:
the stable structure corresponding to the substance related to ginkgo pollen allergy is used as the substance for treating and/or preventing ginkgo pollen allergy; and
the stable structure corresponding to the substance is **characterized by** the structure of said substance that exists in a solid state at a high temperature of 500 °C or higher.

118. The method according to claim 117, **characterized in that** said stable structure corresponding to the substance related to the allergic disease is selected from one or more of the following:
(1) the stable structure corresponding to the substance that causes ginkgo pollen allergy in the organism;
(2) the stable structure corresponding to the substance of the organism itself and related to ginkgo pollen allergy;
(3) the stable structure corresponding to the substance that inhibits the structure that causes ginkgo pollen allergy;
(4) the stable structure corresponding to the substance containing a substance of any one of (1) to (3) above.

119. A substance for treating and/or preventing ginkgo pollen allergy designed according to the method of any one of claims 117-118.

120. The preparation method of the substance for treating and/or preventing ginkgo pollen allergy according to claim 119, **characterized in that** the stable structure corresponding to the substance is prepared by a high-temperature carbonization method.

121. A method for designing substances for treating and/or preventing cat hair allergy, **characterized in that**:
the stable structure corresponding to the substance related to cat hair allergy is used as the substance for treating and/or preventing cat hair allergy; and
the stable structure corresponding to the substance is **characterized by** the structure of said substance that exists in a solid state at a high temperature of 500 °C or higher.

122. The method according to claim 121, **characterized in that** said stable structure corresponding to the substance related to the allergic disease is selected from one or more of the following:
(1) the stable structure corresponding to the substance that causes cat hair allergy in the organism;
(2) the stable structure corresponding to the substance of the organism itself and related to cat hair allergy;
(3) the stable structure corresponding to the substance that inhibits the structure that causes cat hair allergy;
(4) the stable structure corresponding to the substance containing a substance of any one of (1) to (3) above.

123. A substance for treating and/or preventing cat hair allergy designed according to the method of any one of claims 121-122.

124. The preparation method of the substance for treating and/or preventing cat hair allergy according to claim 123, **characterized in that** the stable structure corresponding to the substance is prepared by a high-temperature carbonization method.

125. A method for designing substances for treating and/or preventing dog hair allergy, **characterized in that**:
the stable structure corresponding to the substance related to dog hair allergy is used as the substance for treating and/or preventing dog hair allergy; and
the stable structure corresponding to the substance is **characterized by** the structure of said substance that exists in a solid state at a high temperature of 500 °C or higher.

126. The method according to claim 125, **characterized in that** said stable structure corresponding to the substance related to the allergic disease is selected from one or more of the following:
(1) the stable structure corresponding to the substance that causes dog hair allergy in the organism;
(2) the stable structure corresponding to the substance of the organism itself and related to dog hair allergy;
(3) the stable structure corresponding to the substance that inhibits the structure that causes dog hair allergy;
(4) the stable structure corresponding to the substance containing a substance of any one of (1) to (3) above.

127. A substance for treating and/or preventing dog hair allergy designed according to the method of any one of claims 125-126.

128. The preparation method of the substance for treating and/or preventing dog hair allergy according to claim 127, **characterized in that** the stable structure corresponding to the substance is prepared by a high-temperature carbonization method.

129. A method for designing substances for treating and/or preventing cockroach allergy, **characterized in that**:
the stable structure corresponding to the substance related to cockroach allergy is used as the substance for treating and/or preventing cockroach allergy; and
the stable structure corresponding to the substance is **characterized by** the structure of said substance that exists in a solid state at a high temperature of 500 °C or higher.

130. The method according to claim 129, **characterized in that** said stable structure corresponding to the substance related to the allergic disease is selected from one or more of the following:
(1) the stable structure corresponding to the substance that causes cockroach allergy in the organism;
(2) the stable structure corresponding to the substance of the organism itself and related to cockroach allergy;
(3) the stable structure corresponding to the substance that inhibits the structure that causes cockroach allergy;
(4) the stable structure corresponding to the substance containing a substance of any one of (1) to (3) above.

131. A substance for treating and/or preventing cockroach allergy designed according to the method of any one of claims 129-130.

132. The preparation method of the substance for treating and/or preventing cockroach allergy according to claim 131, **characterized in that** the stable structure corresponding to the substance is prepared by a high-temperature carbonization method.

133. A method for designing substances for treating and/or preventing drug allergy, **characterized in that**:
the stable structure corresponding to the substance related to drug allergy is used as the substance for treating and/or preventing drug allergy; and
the stable structure corresponding to the substance is **characterized by** the structure of said substance that exists in a solid state at a high temperature of 500 °C or higher.

134. The method according to claim 133, **characterized in that** said stable structure corresponding to the substance related to the allergic disease is selected from one or more of the following:
(1) the stable structure corresponding to the substance that causes drug allergy in the organism;
(2) the stable structure corresponding to the substance of the organism itself and related to drug allergy;
(3) the stable structure corresponding to the substance that inhibits the structure that causes drug allergy;
(4) the stable structure corresponding to the substance containing a substance of any one of (1) to (3) above.

135. A substance for treating and/or preventing drug allergy designed according to the method of any one of claims 133-134.

136. The preparation method of the substance for treating and/or preventing drug allergy according to claim 135, **characterized in that** the stable structure corresponding to the substance is prepared by a high-temperature carbonization method.

137. A method for designing substances for treating and/or preventing antibiotics allergy, **characterized in that**:
the stable structure corresponding to the substance related to antibiotics allergy is used as the substance for treating and/or preventing antibiotics allergy; and
the stable structure corresponding to the substance is **characterized by** the structure of said substance that exists in a solid state at a high temperature of 500 °C or higher.

138. The method according to claim 137, **characterized in that** said stable structure corresponding to the substance related to the allergic disease is selected from one or more of the following:
(1) the stable structure corresponding to the substance that causes antibiotics allergy in the organism;
(2) the stable structure corresponding to the substance of the organism itself and related to antibiotics allergy;
(3) the stable structure corresponding to the substance that inhibits the structure that causes antibiotics allergy;
(4) the stable structure corresponding to the substance containing a substance of any one of (1) to (3) above.

139. A substance for treating and/or preventing antibiotics allergy designed according to the method of any one of claims 137-138.

140. The preparation method of the substance for treating and/or preventing antibiotics allergy according to claim 139, **characterized in that** the stable structure corresponding to the substance is prepared by a high-temperature carbonization method.

141. A method for designing substances for treating and/or preventing anesthetic allergy, **characterized in that**:
the stable structure corresponding to the substance related to anesthetic allergy is used as the substance for treating and/or preventing anesthetic allergy; and
the stable structure corresponding to the substance is **characterized by** the structure of said substance that exists in a solid state at a high temperature of 500 °C or higher.

142. The method according to claim 141, **characterized in that** said stable structure corresponding to the substance related to the allergic disease is selected from one or more of the following:
(1) the stable structure corresponding to the substance that causes anesthetic allergy in the organism;
(2) the stable structure corresponding to the substance of the organism itself and related to anesthetic allergy;
(3) the stable structure corresponding to the substance that inhibits the structure that causes anesthetic allergy;
(4) the stable structure corresponding to the substance containing a substance of any one of (1) to (3) above.

143. A substance for treating and/or preventing anesthetic allergy designed according to the method of any one of claims 141-142.

144. The preparation method of the substance for treating and/or preventing anesthetic allergy according to claim 143, **characterized in that** the stable structure corresponding to the substance is prepared by a high-temperature carbonization method.

145. A method for designing substances for treating and/or preventing sulfonamides allergy, **characterized in that**:
the stable structure corresponding to the substance related to sulfonamides allergy is used as the substance for treating and/or preventing sulfonamides allergy; and
the stable structure corresponding to the substance is **characterized by** the structure of said substance that exists in a solid state at a high temperature of 500 °C or higher.

146. The method according to claim 145, **characterized in that** said stable structure corresponding to the substance related to the allergic disease is selected from one or more of the following:
(1) the stable structure corresponding to the substance that causes sulfonamides allergy in the organism;
(2) the stable structure corresponding to the substance of the organism itself and related to sulfonamides allergy;
(3) the stable structure corresponding to the substance that inhibits the structure that causes sulfonamides allergy;
(4) the stable structure corresponding to the substance containing a substance of any one of (1) to (3) above.

147. A substance for treating and/or preventing sulfonamides allergy designed according to the method of any one of claims 145-146.

148. The preparation method of the substance for treating and/or preventing sulfonamides allergy according to claim 147, **characterized in that** the stable structure corresponding to the substance is prepared by a high-temperature carbonization method.

149. A method for designing substances for treating and/or preventing chemical/chemical raw materials allergy, **characterized in that**:
the stable structure corresponding to the substance related to chemical/chemical raw materials allergy is used as the substance for treating and/or preventing chemical/chemical raw materials allergy; and
the stable structure corresponding to the substance is **characterized by** the structure of said substance that exists in a solid state at a high temperature of 500 °C or higher.

150. The method according to claim 149, **characterized in that** said stable structure corresponding to the substance related to the allergic disease is selected from one or more of the following:
(1) the stable structure corresponding to the substance that causes chemical/chemical raw materials allergy in the organism;
(2) the stable structure corresponding to the substance of the organism itself and related to chemical/chemical raw materials allergy;
(3) the stable structure corresponding to the substance that inhibits the structure that causes chemical/chemical raw materials allergy;
(4) the stable structure corresponding to the substance containing a substance of any one of (1) to (3) above.

151. A substance for treating and/or preventing chemical/chemical raw materials allergy designed according to the method of any one of claims 149-150.

152. The preparation method of the substance for treating and/or preventing chemical/chemical raw materials allergy according to claim 151, **characterized in that** the stable structure corresponding to the substance is prepared by a high-temperature carbonization method.

153. A method for designing substances for treating and/or preventing Thiuram mix allergy, **characterized in that**:
the stable structure corresponding to the substance related to Thiuram mix allergy is used as the substance for treating and/or preventing Thiuram mix allergy; and
the stable structure corresponding to the substance is **characterized by** the structure of said substance that exists in a solid state at a high temperature of 500 °C or higher.

154. The method according to claim 53, **characterized in that** said stable structure corresponding to the substance related to the allergic disease is selected from one or more of the following:
(1) the stable structure corresponding to the substance that causes Thiuram mix allergy in the organism;
(2) the stable structure corresponding to the substance of the organism itself and related to Thiuram mix allergy;
(3) the stable structure corresponding to the substance that inhibits the structure that causes Thiuram mix allergy;
(4) the stable structure corresponding to the substance containing a substance of any one of (1) to (3) above.

155. A substance for treating and/or preventing Thiuram mix allergy designed according to the method of any one of claims 153-154.

156. The preparation method of the substance for treating and/or preventing Thiuram mix allergy according to claim 155, **characterized in that** the stable structure corresponding to the substance is prepared by a high-temperature carbonization method.

157. A method for designing substances for treating and/or preventing formaldehyde allergy, **characterized in that**:
the stable structure corresponding to the substance related to formaldehyde allergy is used as the substance for treating and/or preventing formaldehyde allergy; and
the stable structure corresponding to the substance is **characterized by** the structure of said substance that exists in a solid state at a high temperature of 500 °C or higher.

158. The method according to claim 157, **characterized in that** said stable structure corresponding to the substance related to the allergic disease is selected from one or more of the following:
(1) the stable structure corresponding to the substance that causes formaldehyde allergy in the organism;
(2) the stable structure corresponding to the substance of the organism itself and related to formaldehyde allergy;
(3) the stable structure corresponding to the substance that inhibits the structure that causes formaldehyde allergy;
(4) the stable structure corresponding to the substance containing a substance of any one of (1) to (3) above.

159. A substance for treating and/or preventing formaldehyde allergy designed according to the method of any one of claims 157-158.

160. The preparation method of the substance for treating and/or preventing formaldehyde allergy according to claim 159, **characterized in that** the stable structure corresponding to the substance is prepared by a high-temperature carbonization method.

161. A method for designing substances for treating and/or preventing spice mixture allergy, **characterized in that**:
the stable structure corresponding to the substance related to spice mixture allergy is used as the substance for treating and/or preventing spice mixture allergy; and
the stable structure corresponding to the substance is **characterized by** the structure of said substance that exists in a solid state at a high temperature of 500 °C or higher.

162. The method according to claim 161, **characterized in that** said stable structure corresponding to the substance related to the allergic disease is selected from one or more of the following:
(1) the stable structure corresponding to the substance that causes spice mixture allergy in the organism;
(2) the stable structure corresponding to the substance of the organism itself and related to spice mixture allergy;
(3) the stable structure corresponding to the substance that inhibits the structure that causes spice mixture allergy;
(4) the stable structure corresponding to the substance containing a substance of any one of (1) to (3) above.

163. A substance for treating and/or preventing spice mixture allergy designed according to the method of any one of claims 161-162.

164. The preparation method of the substance for treating and/or preventing spice mixture allergy according to claim 163, **characterized in that** the stable structure corresponding to the substance is prepared by a high-temperature carbonization method.

165. A method for designing substances for treating and/or preventing fragrance mix allergy, **characterized in that**:
the stable structure corresponding to the substance related to fragrance mix allergy is used as the substance for treating and/or preventing fragrance mix allergy; and
the stable structure corresponding to the substance is **characterized by** the structure of said substance that exists in a solid state at a high temperature of 500 °C or higher.

166. The method according to claim 165, **characterized in that** said stable structure corresponding to the substance related to the allergic disease is selected from one or more of the following:
(1) the stable structure corresponding to the substance that causes fragrance mix allergy in the organism;
(2) the stable structure corresponding to the substance of the organism itself and related to fragrance mix allergy;
(3) the stable structure corresponding to the substance that inhibits the structure that causes fragrance mix allergy;
(4) the stable structure corresponding to the substance containing a substance of any one of (1) to (3) above.

167. A substance for treating and/or preventing fragrance mix allergy designed according to the method of any one of claims 165-166.

168. The preparation method of the substance for treating and/or preventing fragrance mix allergy according to claim 167, **characterized in that** the stable structure corresponding to the substance is prepared by a high-temperature carbonization method.

169. A method for designing substances for treating and/or preventing Balsam of Peru allergy, **characterized in that**:
the stable structure corresponding to the substance related to Balsam of Peru allergy is used as the substance for treating and/or preventing Balsam of Peru allergy; and
the stable structure corresponding to the substance is **characterized by** the structure of said substance that exists in a solid state at a high temperature of 500 °C or higher.

170. The method according to claim 169, **characterized in that** said stable structure corresponding to the substance related to the allergic disease is selected from one or more of the following:
(1) the stable structure corresponding to the substance that causes Balsam of Peru allergy in the organism;
(2) the stable structure corresponding to the substance of the organism itself and related to Balsam of Peru allergy;
(3) the stable structure corresponding to the substance that inhibits the structure that causes Balsam of Peru allergy;
(4) the stable structure corresponding to the substance containing a substance of any one of (1) to (3) above.

171. A substance for treating and/or preventing Balsam of Peru allergy designed according to the method of any one of claims 169-170.

172. The preparation method of the substance for treating and/or preventing Balsam of Peru allergy according to claim 171, **characterized in that** the stable structure corresponding to the substance is prepared by a high-temperature carbonization method.

173. A method for designing substances for treating and/or preventing lanolin alcohol allergy, **characterized in that**:
the stable structure corresponding to the substance related to lanolin alcohol allergy is used as the substance for treating and/or preventing lanolin alcohol allergy; and
the stable structure corresponding to the substance is **characterized by** the structure of said substance that exists in a solid state at a high temperature of 500 °C or higher.

174. The method according to claim 173, **characterized in that** said stable structure corresponding to the substance related to the allergic disease is selected from one or more of the following:
(1) the stable structure corresponding to the substance that causes lanolin alcohol allergy in the organism;
(2) the stable structure corresponding to the substance of the organism itself and related to lanolin alcohol allergy;
(3) the stable structure corresponding to the substance that inhibits the structure that causes lanolin alcohol allergy;
(4) the stable structure corresponding to the substance containing a substance of any one of (1) to (3) above.

175. A substance for treating and/or preventing lanolin alcohol allergy designed according to the method of any one of claims 173-174.

176. The preparation method of the substance for treating and/or preventing lanolin alcohol allergy according to claim 175, **characterized in that** the stable structure corresponding to the substance is prepared by a high-temperature carbonization method.

177. A method for designing substances for treating and/or preventing chlorides allergy, **characterized in that**:
the stable structure corresponding to the substance related to chlorides allergy is used as the substance for treating and/or preventing chlorides allergy; and
the stable structure corresponding to the substance is **characterized by** the structure of said substance that exists in a solid state at a high temperature of 500 °C or higher.

178. The method according to claim 177, **characterized in that** said stable structure corresponding to the substance related to the allergic disease is selected from one or more of the following:
(1) the stable structure corresponding to the substance that causes chlorides allergy in the organism;
(2) the stable structure corresponding to the substance of the organism itself and related to chlorides allergy;
(3) the stable structure corresponding to the substance that inhibits the structure that causes chlorides allergy;
(4) the stable structure corresponding to the substance containing a substance of any one of (1) to (3) above.

179. A substance for treating and/or preventing chlorides allergy designed according to the method of any one of claims 177-178.

180. The preparation method of the substance for treating and/or preventing chlorides allergy according to claim 179, **characterized in that** the stable structure corresponding to the substance is prepared by a high-temperature carbonization method.

181. Use of the substance according to any one of claims 3, 7, 11, 15, 19, 23, 27, 31, 35, 39, 43, 47, 51, 55, 59, 63, 67, 71, 75, 79, 83, 87, 91, 95, 99, 103, 107, 111, 115, 119, 123, 131, 135, 139, 143, 147, 151, 155, 159, 163, 167, 171, 175 and 179 in the preparation of medicines, health products, foods, and food additives.
